# EUROPEAN PATENT APPLICATION

(11) **EP 4 763 280 A1**
(43) Date of publication of application: **24.06.2026**
(21) Application number: 24221962.4
(22) Date of filing: 19.12.2024
(51) Int. Cl.: A61P 9/04, A61P 9/06, A61P 9/10, A61P 9/12, A61P 29/00, A61P 31/04, A61P 31/10, A61P 31/12, A61P 33/00, A61P 35/00, A61P 37/02, C07D 498/04, A61K 31/4162

(54) **PHARMACEUTICALLY ACTIVE PYRAZOLO[5,1-C][1,4]BENZOXAZEPINE DERIVATIVES**

(71) Applicant: Lead Discovery Center GmbH, 44227 Dortmund (DE)
(72) Inventor: Di Lucrezia, Raffaella, 42277 Wuppertal (DE); Choidas, Axel, 58313 Herdecke (DE); Klebl, Bert M., 44229 Dortmund (DE); Nussbaumer, Peter, 44227 Dortmund (DE); Dinkel, Klaus, 67705 Trippstadt (DE)
(74) Representative: Arth, Hans-Lothar

(57) **Abstract**

The present invention relates to novel pyrazolo[5,1-c][1,4]benzoxazepine derivatives of general formula (I) and stereoisomeric forms, hydrates, solvates and pharmaceutically acceptable salts thereof, as well as pharmaceutical compositions containing at least one pyrazolo[5,1-c][1,4]benzoxazepine derivative together with at least one pharmaceutically acceptable carrier, excipient and/or diluent. Said pyrazolo[5,1-c][1,4]benzoxazepine derivatives are particularly useful for the treatment and/or prophylaxis of proliferative diseases, immunological disorders, infectious diseases, autoimmune diseases, inflammatory disorders and circulatory diseases.

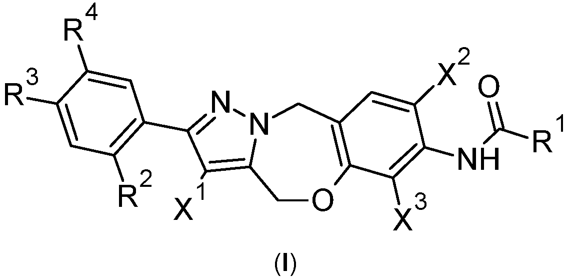

## Description

The present invention relates to novel pyrazolo[5,1-c][1,4]benzoxazepine derivatives of general formula (I) and stereoisomeric forms, hydrates, solvates and pharmaceutically acceptable salts thereof, as well as pharmaceutical compositions containing at least one pyrazolo[5,1-c][1,4]benzoxazepine derivative together with at least one pharmaceutically acceptable carrier, excipient and/or diluent. Said pyrazolo[5,1-c][1,4]benzoxazepine derivatives are particularly useful for the treatment and/or prophylaxis of proliferative diseases, immunological disorders, infectious diseases, autoimmune diseases, inflammatory disorders and circulatory diseases.

### Background of the invention

Neutrophils are one of the first cells of the immune system recruited to the site of infection, representing the host's most effective and numerous front-line defenders. Recently, a novel antimicrobial mechanism of neutrophils has been described: upon activation, they release DNA and a subset of their granule content, forming neutrophil extracellular traps (NETs). These extracellular, web-like, chromatin structures, which contain histones and neutrophil granule proteins, can trap and kill a broad spectrum of microbes, including Gram-positive and Gram-negative bacteria, fungi, protozoa and viruses. NETs are formed during active cell death (NETosis) according to different mechanisms depending on the stimulus to which neutrophils are exposed. NETosis differs significantly from known types of cell death, including apoptosis and necrosis. Thus, NETs have a high cytotoxic potential and may directly contribute to host cell death and chronic tissue damage when formed in excess (Grabcanovic-Musija et al. Respiratory Research 2015, 16, 59).

NETs are diversely involved in the innate immune system (Grayson et al, J. Leukoc Biol. 2016, 99(2), 253-264), such as protein-DNA complexes within NETs (e.g., LL-37/DNA) activate plasmacytoid dendritic cells to synthesize T1IFNs. NETs interact with B and T lymphocytes, linking the innate and adaptive immune systems and promoting their activation. NETs can further activate platelets and coagulation factors and promote thrombosis (Kambas K. et al. Front. Immunol. 2012, 3, 385.), rendering the targeting of NET formation to prevent arterial or venous thrombosis as could have therapeutically efficient (Martinod K. et al. Blood 2014, 123, 2768-2776). NET contents are potentially toxic to endothelial cells, promoting vasculopathy and apoptosis through matrix metalloproteinases and histones. Several amplification loops of NETosis exist, whereby products found within NETs (autoantigens, complement activating factors, proinflammatory cytokines) or antibodies and immune complexes to/containing NET-related proteins stimulate neutrophils to undergo NET formation. Also, in a feed-forward loop, NET proteins stimulate the inflammasome (NLRP3) in macrophages with resultant release of inflammatory cytokines (IL-1 and IL-18) that promote additional NET formation in granulocytes.

Neutrophil-mediated immunogenicity may occur due to enhanced NET formation and impairments in degradation of NETs. Among patients with systemic lupus erythematosus (SLE), impaired NET degradation has been associated with lupus nephritis, hypocomplementemia, production of T1IFNs, and elevated autoantibody levels. In addition to SLE, impairments in NET degradation have been reported in association with ANCA-associated vasculitis, antiphospholipid antibody syndrome, and drug-induced vasculitis. In each of these conditions, defects in clearance of NETs may allow NETs to persist longer in vivo and thereby facilitate NET-mediated immunogenic and pathogenic effects. Moreover, neutrophils and NETs infiltrate human and murine atherosclerotic plaques, indicating a critical role of NET formation in the pathogenesis of atherosclerosis.

Despite the fact that neutrophils have various types of granules containing hundreds of proteins, only a limited number of proteins (20-30) have been detected within NETs. Many of the proteins found within NETs represent the major autoantigenic targets in rheumatologic diseases: SLE (dsDNA, histones), ANCA-associated vasculitis (myeloperoxidase, proteinase 3), and rheumatoid arthritis (vimentin, enolase, histones). Novel autoantibodies to NET-related proteins have been described in association with various autoimmune diseases. Cathelicidin and matrix metalloproteinase 9 have been demonstrated within NETs, and autoantibodies to these proteins have been identified in patients with SLE and characterized as immunogenic and vasculopathic. Protein cargo within NETs may be influenced directly by the nature of the inciting stimuli. In rheumatoid arthritis, disease-associated autoantibodies induced a wider array of NET-protein content compared with NET induction with proinflammatory cytokines.

Moreover, neutrophil extracellular traps can further activate inflammasomes, thereby representing another important amplifier of inflammatory pathways. Both NETs and cathelicidins (LL-37, protein contained in NETs) can activate caspase-1, the central enzyme of the inflammasome, in human and murine macrophages. Activated inflammasome leads to maturation/activation and release of active inflammatory cytokines IL-1β and IL-18, and these effects are enhanced in macrophages derived from patients with SLE (Kahlenberg J. M., et al. J. Immunol. 2013, 190, 1217-1226).

Besides the release of IL-1β and IL-18 activated inflammatory caspases, i.e. caspase-1, caspase-4, caspase-5 (humans) and caspase-11 (mice) can cleave mouse and human gasdermin D (GSDMD) at aspartic acid D275/D276, wherein the formation of plasma membrane pores by oligomerization of gasdermin D is induced, resulting in lytic cell death called pyroptosis.

International patent application WO 2023/287793 A1 discloses pyrazolo[5,1-c][1,4]benzoxazepine compounds as agonists of glycolytic enzyme phosphofructokinase-1 liver type and are useful for treating diseases associated with the activity of glycolytic enzyme phosphofructokinase-1 liver type, such as cancer, diabetes, sepsis, and septic shock. The compounds, particularly LDC7559 and NA-11 (example 6) inhibit NET formation (NETosis) independent of GSDMD, but do not inhibit GSDMD-dependent pyroptosis. In primary human monocytes and human monocytic THP-1 cells inhibition of GSDMD-dependent pyroptosis was not observed. Amara et al. (Cell 2021, 184, 4480-4494) confirmed that these pyrazolo[5,1-c][1,4]benzoxazepine compounds do not inhibit NETosis by compromising the MEK-ERK signaling cascade downstream of PDE6d. A binding of NA-11 to GSDMD could not be observed. Consequently, the compounds of WO 2023/287793 A1 do not inhibit gasdermin pore formation and do not inhibit GSDMD-driven pyroptosis.

These pore-forming gasdermins are extremely hazardous to the cell, and inappropriate activation is already associated with deafness and alopecia as well as exacerbation of sepsis in animal models. Mutation of Gsdma3 causes epidermal hyperplasia, hyperkeratosis, and hair loss in mice. Gsdma3-deficient mice show normal hair development, and heterozygous Gsdma3 mutation causes the same alopecia phenotype. These observations suggest that alopecia is caused by a gain-of-function mutation in Gsdma3. Consistent with the constitutive pyroptosis induced by the mutant proteins, strong inflammatory responses in the skin and gradual depletion of the skin bulge stem cells are observed in Gsdma3-mutant mice. Polymorphisms of GSDMB in humans are strongly associated with early childhood asthma, a disease characterized by chronic inflammation. A potential role of GSDMB and GSDMC in cancer progression has also been reported. Furthermore DFNA5-mediated pyroptosis in the cochlear system causes hearing loss. DFNA5 shares the biochemical properties of GSDMD, the deafness mutant should have spontaneous pyroptosis-inducing activity due to the loss of the gasdermin-C domain. Consistent with the gain of function of the deafness mutation, DFNA5 knockout mice exhibit no hearing impairment.

It is the objective of the present invention to provide compounds and/or pharmaceutically acceptable salts thereof which inhibit the formation of neutrophil extracellular traps (NET) and inhibit the GSDMD-driven pyroptosis, thus can be used as pharmaceutically active agents, especially for the treatment and/or prophylaxis of proliferative diseases, tumours, cancers, metastases, immunological disorders, autoimmune diseases, inflammatory disorders and circulatory diseases, as well as compositions comprising at least one of those compounds as pharmaceutically active agents.

The objective of the present invention is solved by the teaching of the independent claims. Further advantageous features, aspects and details of the invention are evident from the dependent claims, the description, the figures, and the examples of the present application.

### Description of the invention

The inventors have found that pyrazolo[5,1-c][1,4]benzoxazepine compounds of formula (I) bearing either (i) a chloro substituent at the pyrazolo ring or (ii) an ortho-chloro or ortho-methyl substituent at the adjacent phenyl ring are potent inhibitors of GSDMD activity and also exhibit NET formation inhibition activity. Thus, the present invention is directed to a compound of general formula **(I)** 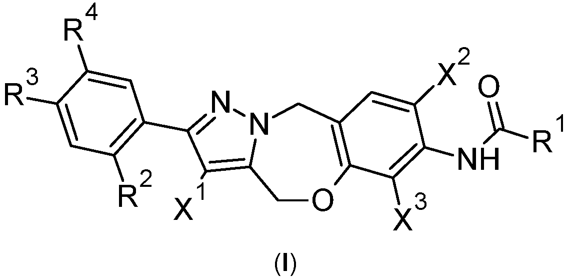 wherein
X¹ represents -H -CH₃, -C₂H₅, -C₃H₇, -CH(CH₃)₂, -cyclo-C₃H₅, -F, -Cl, -Br, or -CF₃ ;
X² and X³ are independently of each other selected from -H, -F, -Cl, and -Br;
R¹ represents -CH₃, -C₂H₅, -C₃H₇, -CH(CH₃)₂, or -cyclo-C₃H₅ ;
R² represents -H, -F, -Cl, -CH₃, -C₂H₅, -C₃H₇, -CH(CH₃)₂, -CF₃, -OCF₃, -CN, -OH, or -OCH₃ ; preferably R² represents -H, -F, -Cl, -CH₃, -C₂H₅, -C₃H₇, -CF₃, -OCF₃, -CN, -OH, or -OCH₃ ; more preferably R² represents -H, -F, -Cl, -CH₃, -C₂H₅, -CF₃, -OCF₃, -CN, -OH, or -OCH₃ ; still more preferably R² represents -H, -F, -Cl, -CH₃, -CF₃, -OCF₃, -CN, -OH, or -OCH₃ ;
R³ and R⁴ are independently of each other selected from -H, -F, -CH₃, and -CF₃ ; with the proviso that when X¹, X², and X³ are simultaneously -H, R² represents -Cl, -C₂H₅, -C₃H₇, -CH(CH₃)₂, -CF₃, or -OCF₃ ; preferably R² represents -Cl, -C₂H₅, -C₃H₇, -CF₃, or -OCF₃ ; more preferably R² represents -Cl, -C₂H₅, -CF₃ or -OCF₃ ; still more preferably R² represents -Cl, -CF₃ or -OCF₃ ;
or enantiomers, mixtures of enantiomers, diastereoisomers, mixtures of diastereoisomers, hydrates, solvates, racemates or pharmaceutically acceptable salts thereof.

International patent application WO 2023/287793 A1 discloses pyrazolo[5,1-c][1,4]benzoxazepine compounds as agonists of glycolytic enzyme phosphofructokinase-1 liver type and are useful for treating diseases associated with the activity of glycolytic enzyme phosphofructokinase-1 liver type, such as cancer, diabetes, sepsis, and septic shock. The compounds, particularly LDC7559 and NA-11 (example 6 in WO 2023/287793 A1) inhibit NET formation (NETosis) independent of GSDMD, but do not inhibit GSDMD-dependent pyroptosis. In primary human monocytes and human monocytic THP-1 cells inhibition of GSDMD-dependent pyroptosis was not observed. Amara et al. (Cell 2021, 184, 4480-4494) confirmed that these pyrazolo[5,1-c][1,4]benzoxazepine compounds do not inhibit NETosis by compromising the MEK-ERK signaling cascade downstream of PDE6d. A binding of NA-11 to GSDMD could not be observed. Thus, the pyrazolo[5,1-c][1,4]benzoxazepine compounds of the prior art are not potent inhibitors of GSDMD activity.

The inventors could show that LDC7559 exhibited only a weak inhibition in assay (IC₅₀ = 3.49 µM) and NA-11 showed no binding (IC₅₀ > 23 µM, see **Table 3**). Consequently, the pyrazolo[5,1-c][1,4]benzoxazepine compounds of WO 2023/287793 A1 only inhibit NETosis, but not GSDMD activity, whereas the inventive compounds of formula **(I)** potently inhibit GSDMD as well as NETosis. Experimental data provided herein demonstrate a correlation between the activity of the inventive compounds in NET formation assays and their inhibition of GSDMD N-terminal (GSDMD-NT) activity. However, no correlation is observed between NET formation and reactive oxygen species (ROS) assays. This distinction underscores the selectivity of these compounds for GSDMD-mediated pathways over ROS-dependent mechanisms. In contrast, the compounds of WO 2023/287793 A1 may exhibit activity on targets other than GSDMD, such as PFKL since these compounds exhibited only a minimal activity in GSDMD-NT assays.

Therefore, the present invention is directed to a compound of general formula **(I),** wherein
X¹ represents -H -CH₃, -C₂H₅, -C₃H₇, -CH(CH₃)₂, -cyclo-C₃H₅, -F, -Cl, -Br, or -CF₃ ;
X² and X³ are independently of each other selected from -H, -F, -Cl, and -Br;
R¹ represents -CH₃, -C₂H₅, -C₃H₇, -CH(CH₃)₂, or -cyclo-C₃H₅ ;
R² represents -H, -F, -Cl, -CH₃, -C₂H₅, -C₃H₇, -CH(CH₃)₂, -CF₃, -OCF₃, -CN, -OH, or -OCH₃ ;
R³ and R⁴ are independently of each other selected from -H, -F, -CH₃, and -CF₃ ;
with the proviso that at least one of X¹ and R² is -Cl, -CH₃, or -CF₃,
or enantiomers, mixtures of enantiomers, diastereoisomers, mixtures of diastereoisomers, hydrates, solvates, racemates or pharmaceutically acceptable salts thereof.

A preferred embodiment according to the present invention relates to an inventive compound of general formula **(I),** wherein
X¹ represents -H -CH₃, -C₂H₅, -C₃H₇, -CH(CH₃)₂, -cyclo-C₃H₅, -F, -Cl, -Br, or -CF₃ ;
X² and X³ are independently of each other selected from -H, -F, -Cl, and -Br;
R¹ represents -CH₃, -C₂H₅, -C₃H₇, -CH(CH₃)₂, or -cyclo-C₃H₅ ;
R² represents -H, -F, -Cl, -CH₃, -CF₃, -OCF₃, -CN, -OH, or -OCH₃ ;
R³ and R⁴ are independently of each other selected from -H, -F, -CH₃, and -CF₃ ; with the proviso that when X¹, X², and X³ are simultaneously -H, R² represents -Cl, -CF₃, or -OCF₃ ;
or enantiomers, mixtures of enantiomers, diastereoisomers, mixtures of diastereoisomers, hydrates, solvates, racemates or pharmaceutically acceptable salts thereof.

A preferred embodiment according to the present invention relates to an inventive compound of general formula **(I),** wherein
X¹ represents -H -CH₃, -C₂H₅, -C₃H₇, -CH(CH₃)₂, -cyclo-C₃H₅, -F, -Cl, -Br, or -CF₃ ;
X² and X³ are independently of each other selected from -H, -F, -Cl, and -Br;
R¹ represents -CH₃, -C₂H₅, -C₃H₇, -CH(CH₃)₂, or -cyclo-C₃H₅ ;
R² represents -H, -F, -Cl, -CH₃, -C₂H₅, -C₃H₇, -CH(CH₃)₂, -CF₃, -OCF₃, -CN, -OH, or -OCH₃
R³ and R⁴ represent -H, with the proviso that when X¹, X², and X³ are simultaneously -H, R² represents -Cl, -CF₃ or -OCF₃ ;
or enantiomers, mixtures of enantiomers, diastereoisomers, mixtures of diastereoisomers, hydrates, solvates, racemates or pharmaceutically acceptable salts thereof.

A preferred embodiment according to the present invention relates to an inventive compound of general formula **(I),** wherein X² and X³ represent -H and R¹ - R⁴, and X¹ have the meaning as defined above and with the proviso that
when X¹, X², and X³ are simultaneously -H, R² represents -Cl, -CF₃ or -OCF₃.

A preferred embodiment according to the present invention relates to an inventive compound of general formula **(I),** wherein X², X³, R³, and R⁴ represent -H and R¹, R², and X¹ have the meaning as defined above and with the proviso that
with the proviso that when X¹, X², and X³ are simultaneously -H, R² represents -Cl, -CF₃, or -OCF₃.

Another preferred embodiment according to the present invention relates to an inventive compound of general formula **(I),** X¹ represents -H, -CH₃, -cyclo-C₃H₅, -F, -Cl, -Br, or -CF₃ ; and X², X³, and R¹ - R⁴ have the meaning as defined above and with the proviso that when X¹, X², and X³ are simultaneously -H, R² represents -Cl, -CF₃, or -OCF₃.

Another preferred embodiment according to the present invention relates to an inventive compound of general formula **(I),** X¹ represents -H, -CH₃, -cyclo-C₃H₅, -F, -Cl, -Br, or -CF₃ ; and X², X³, and R¹ - R⁴ have the meaning as defined above and with the proviso that when X¹ represents -Cl and X² and X³ are-H, R² represents -Cl, -H, -Cl, -CH₃, -CF₃, or -OCF₃.

Another preferred embodiment according to the present invention relates to an inventive compound of general formula **(I),** X¹ represents -H, -CH₃, -cyclo-C₃H₅, -F, -Cl, -Br, or -CF₃ ; and X², X³, and R¹ - R⁴ have the meaning as defined above and with the proviso that when X¹, represents -Cl and X² and X³ are -H, R² represents -H, -Cl, -CH₃, -CF₃, or -OCF₃.

Another preferred embodiment according to the present invention relates to an inventive compound of general formula **(I),** wherein X² represents -Cl.

Another preferred embodiment according to the present invention relates to an inventive compound of general formula **(I),** wherein X³ represents -F.

Another preferred embodiment according to the present invention relates to an inventive compound of general formula **(I),** wherein X² represents -Cl and X³ represents -F.

Another preferred embodiment according to the present invention relates to an inventive compound of general formula **(I),** wherein R¹ represents -CH₃, -C₂H₅, -CH(CH₃)₂, or -cyclo-C₃H₅.

Even more preferred compounds of the present invention are compounds of general formula **(II)** 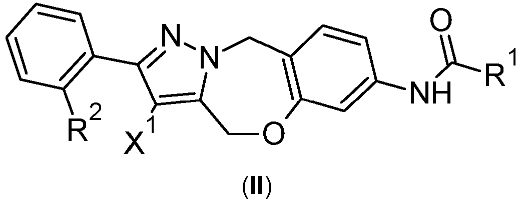 wherein X¹, R¹ and R² have the meanings as defined above, and wherein when X¹ represents -H, R² represents -Cl, -CF₃, or -OCF₃.

A preferred embodiment according to the present invention relates to an inventive compound of general formula **(II),** wherein X¹ represents -H, -CH₃, -cyclo-C₃H₅, -F, -Cl, -Br, or -CF₃ ; and R¹ and R² have the meaning as defined above and with the proviso that when X¹ represents -Cl, R² represents -Cl, -H, -CH₃, -CF₃, or -OCF₃.

A preferred embodiment according to the present invention relates to an inventive compound of general formula **(II),** wherein X¹ represents -H, -CH₃, -cyclo-C₃H₅, -F, -Cl, -Br, or -CF₃ ; and R¹ and R² have the meaning as defined above and with the proviso that when X¹ represents -Cl, R² represents -H, -F, -Cl, -CH₃, -CF₃, or -OCF₃.

Even more preferred compounds of the present invention are compounds of general formula **(II-A)** 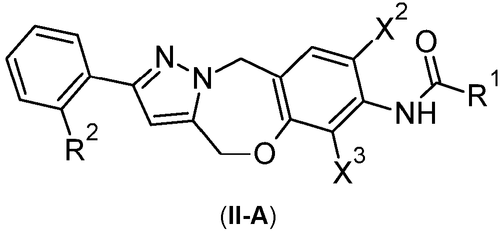 wherein R² X², X³, and R¹ has the meanings as defined above with the proviso that when X² and X³ are simultaneously -H, R² represents -Cl, -CF₃, or -OCF₃ .

Preferred is also a compound of general formula **(II-A),** wherein R² represents -Cl; -CF₃, or -OCF₃ and R¹, X² and X³ have the meanings as defined above.

Preferred is also a compound of general formula **(II-A),** wherein X² represents -Cl.

Preferred is also a compound of general formula **(II-A),** wherein X³ represents -F.

Preferred is also a compound of general formula **(II-A),** wherein X² represents -Cl and X³ represents -F.

Preferred is also a compound of general formula **(II-A),** wherein R¹ represents -CH₃, -C₂H₅, -CH(CH₃)₂, or -cyclo-C₃H₅ .

Also more preferred compounds of the present invention are compounds of general formula **(II-B)** 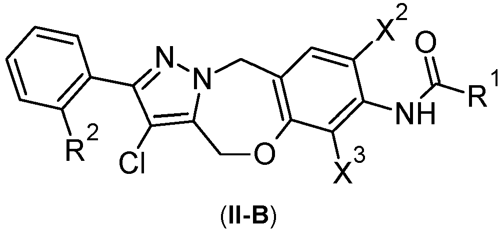 wherein R² represents -H, -F, -Cl, -CH₃, -CF₃, -OCF₃, -CN, -OH, or -OCH₃ ; and X², X³, and R¹ have the meanings as defined above.

Preferred is also a compound of general formula **(II-B),** wherein R² represents -H, -F, -Cl, -CH₃, -CF₃, or -OCF₃ ; and R¹ has the meanings as defined above.

Preferred is also a compound of general formula **(II-B),** wherein R² represents -H, -Cl, -CH₃, -CF₃, or -OCF₃ ; and R¹ has the meanings as defined above.

Preferred is also a compound of general formula **(II-B),** wherein R² represents -Cl; and R¹ have the meanings as defined above.

Preferred is also a compound of general formula **(II-B),** wherein X² represents -Cl.

Preferred is also a compound of general formula **(II-B),** wherein X³ represents -F.

Preferred is also a compound of general formula **(II-B),** wherein X² represents -Cl and X³ represents -F.

Preferred is also a compound of general formula **(II-B),** wherein R¹ represents -CH₃, -C₂H₅, -CH(CH₃)₂, or -cyclo-C₃H₅ .

Also more preferred compounds of the present invention are compounds of general formula **(II-C)** 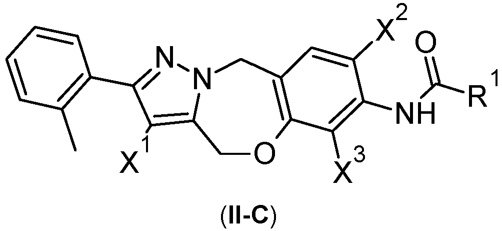 wherein X¹ represents -CH₃, -C₂H₅, -C₃H₇, -CH(CH₃)₂, -cyclo-C₃H₅, -F, -Cl, -Br, or -CF₃, and X², X³, and R¹ have the meanings as defined above.

Preferred is also a compound of general formula **(II-C),** wherein X¹ represents -CH₃, -cyclo-C₃H₅, -F, -Cl, -Br, and X², X³, and R¹ have the meanings as defined above..

Preferred is also a compound of general formula **(II-C),** wherein X² represents -Cl.

Preferred is also a compound of general formula **(II-C),** wherein X³ represents -F.

Preferred is also a compound of general formula **(II-C),** wherein X² represents -Cl and X³ represents -F.

Preferred is also a compound of general formula **(II-C),** wherein R¹ represents -CH₃, -C₂H₅, -CH(CH₃)₂, or -cyclo-C₃H₅ .

Even more preferred compounds of the present invention are compounds of general formula **(II-D)**

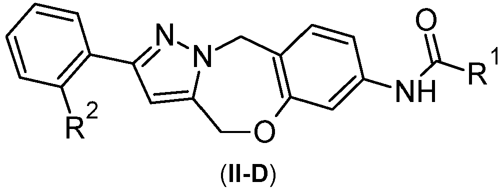

R² represents -Cl, -CF₃, or -OCF₃ ; and R¹ has the meaning as defined above.

Preferred is also a compound of general formula **(II-D),** wherein R² represents -Cl, -CF₃, or -OCF₃ and R¹ has the meanings as defined above.

Preferred is also a compound of general formula **(II-D),** wherein R¹ represents -CH₃, -C₂H₅, -CH(CH₃)₂, or -cyclo-C₃H₅ .

Also more preferred compounds of the present invention are compounds of general formula **(II-E)** 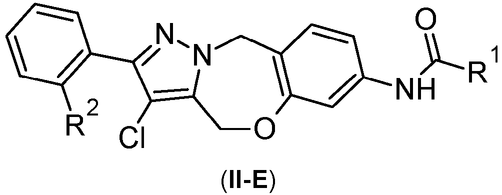 wherein R² represents -H, -F, -Cl, -CH₃, -C₂H₅, -C₃H₇, -CH(CH₃)₂, -CF₃, -OCF₃, -CN, -OH, or -OCH₃, and R¹ has the meaning as defined above.

Preferred is also a compound of general formula **(II-E),** wherein R² represents -H, -F, -Cl, -CH₃, -CF₃, or -OCF₃ ; and R¹ has the meaning as defined above.

Preferred is also a compound of general formula **(II-E),** wherein R² represents -H, -Cl, -CH₃, -CF₃, or -OCF₃ ; and R¹ has the meaning as defined above.

Preferred is also a compound of general formula **(II-E),** wherein R¹ represents -CH₃, -C₂H₅, -CH(CH₃)₂, or -cyclo-C₃H₅ .

Also more preferred compounds of the present invention are compounds of general formula **(II-F)** 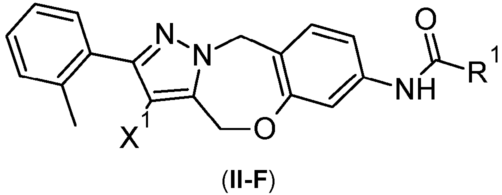 wherein X¹ represents -CH₃, -C₂H₅, -C₃H₇, -CH(CH₃)₂, -cyclo-C₃H₅, -F, -Cl, -Br, or -CF₃, and R¹ has the meanings as defined above.

Preferred is also a compound of general formula **(II-F),** wherein X¹ represents -CH₃, -cyclo-C₃H₅, -F, -Cl, -Br, and R¹ has the meanings as defined above..

Preferred is also a compound of general formula **(II-F),** wherein R¹ represents -CH₃, -C₂H₅, -CH(CH₃)₂, or -cyclo-C₃H₅ .

Preferably, the inventive compounds are selected from:
N-[2-(2-chlorophenyl)-4,10-dihydropyrazolo[5,1-c][1,4]benzoxazepin-7-yl]acetamide,
N-[2-(2-chlorophenyl)-4,10-dihydropyrazolo[5,1-c][1,4]benzoxazepin-7-yl]-2-methyl-propanam ide,
N-[2-(2-chlorophenyl)-4,10-dihydropyrazolo[5,1-c][1,4]benzoxazepin-7-yl]propanamide,
N-[3-chloro-2-(o-tolyl)-4,10-dihydropyrazolo[5,1-c][1,4]benzoxazepin-7-yl]propanamide,
N-[3-chloro-2-(o-tolyl)-4,10-dihydropyrazolo[5,1-c][1,4]benzoxazepin-7-yl]-2-methyl-propanam ide,
N-(3-chloro-2-(2-chlorophenyl)-4H,10H-benzo[f]pyrazolo[5,1-c][1,4]oxazepin-7-yl)isobutyramide,
N-(3-chloro-2-phenyl-4,10-dihydropyrazolo[5,1-c][1,4]benzoxazepin-7-yl)propanamide,
N-(3-chloro-2-phenyl-4,10-dihydropyrazolo[5,1-c][1,4]benzoxazepin-7-yl)-2-methyl-propanam ide,
N-(3-chloro-2-(2-(trifluoromethyl)phenyl)-4H,10H-benzo[f]pyrazolo[5,1-c][1,4]oxazepin-7-yl)cyclopropanecarboxamide,
N-(3-chloro-2-(2-(trifluoromethoxy)phenyl)-4H,10H-benzo[f]pyrazolo[5,1-c][1,4]oxazepin-7-yl)propionamide
N-(3-chloro-2-(2-(trifluoromethoxy)phenyl)-4H,10H-benzo[f]pyrazolo[5,1-c][1,4]oxazepin-7-yl)acetamide,,
N-(3,8-dichloro-2-(2-(trifluoromethoxy)phenyl)-4H,10H-benzo[f]pyrazolo[5,1-c][1,4]oxazepin-7-yl)propionamide,
N-(3-chloro-2-(2-chlorophenyl)-4H,10H-benzo[f]pyrazolo[5,1-c][1,4]oxazepin-7-yl)cyclopropanecarboxamide,
N-(3-chloro-2-(2-fluorophenyl)-4H,10H-benzo[f]pyrazolo[5,1-c][1,4]oxazepin-7-yl)propionamide,
N-(3-chloro-2-(2-fluorophenyl)-4H,10H-benzo[f]pyrazolo[5,1-c][1,4]oxazepin-7-yl)acetamide,
N-(3-chloro-2-(2-(trifluoromethyl)phenyl)-4H,10H-benzo[f]pyrazolo[5,1-c][1,4]oxazepin-7-yl)propionamide,
N-(3-chloro-2-(2-fluorophenyl)-4H,10H-benzo[f]pyrazolo[5,1-c][1,4]oxazepin-7-yl)cyclopropanecarboxamide,
N-(3-chloro-2-(2-fluorophenyl)-4H,10H-benzo[f]pyrazolo[5,1-c][1,4]oxazepin-7-yl)isobutyramide,
N-(3-chloro-2-(2-(trifluoromethoxy)phenyl)-4H,10H-benzo[f]pyrazolo[5,1-c][1,4]oxazepin-7-yl)cyclopropanecarboxamide,
N-(3-chloro-2-(2-chlorophenyl)-4H,10H-benzo[f]pyrazolo[5,1-c][1,4]oxazepin-7-yl)propionamide,
N-(3-chloro-2-(2-chlorophenyl)-4H,10H-benzo[f]pyrazolo[5,1-c][1,4]oxazepin-7-yl)acetamide,
N-(3-chloro-2-(2-(trifluoromethoxy)phenyl)-4H,10H-benzo[f]pyrazolo[5,1 c][1,4]oxazepin-7-yl)isobutyramide,
N-(3-chloro-2-(2-(trifluoromethyl)phenyl)-4H,10H-benzo[f]pyrazolo[5,1-c][1,4]oxazepin-7-yl)isobutyramide,
N-(3-chloro-2-(o-tolyl)-4H,10H-benzo[f]pyrazolo[5,1-c][1,4]oxazepin-7-yl)cyclopropanecarboxamide,
N-(3-chloro-2-(₀-tolyl)-4H,1 OH-benzo[f]pyrazolo[5, 1-c][1 ,4]oxazepin-7 -yl)acetamide,
N-(3,8-dichloro-2-(2-(trifluoromethoxy)phenyl)-4H,10H-benzo[f]pyrazolo[5,1-c][1,4]oxazepin-7-yl)propionamide,
N-[3-cyclopropyl-2-(o-tolyl)-4,10-dihydropyrazolo[5,1-c][1,4]benzoxazepin-7-yl]acetamide,
N-[2-[2-(trifluoromethoxy)phenyl]-3-(trifluoromethyl)-4,10-dihydropyrazolo[5,1-c][1,4]benzoxazepin-7-yl]acetamide,
N-[2-[2-(trifluoromethoxy)phenyl]-3-(trifluoromethyl)-4,10-dihydropyrazolo[5,1-c][1,4]benzoxazepin-7-yl]cyclopropanecarboxamide,
N-[3-bromo-2-[2-(trifluoromethoxy)phenyl]-4,10-dihydropyrazolo[5,1-c][1,4]benzoxazepin-7-yl]propanamide,
N-[3-bromo-2-[2-(trifluoromethoxy)phenyl]-4,10-dihydropyrazolo[5,1-c][1,4]benzoxazepin-7-yl]-2-methyl-propanamide,
N-[3-bromo-2-[2-(trifluoromethoxy)phenyl]-4,10-dihydropyrazolo[5,1-c][1,4]benzoxazepin-7-yl]cyclopropanecarboxamide,
N-[3-methyl-2-(o-tolyl)-4,10-dihydropyrazolo[5,1-c][1,4]benzoxazepin-7-yl]cyclopropanecarboxam ide,
N-[3-bromo-2-(2-methoxyphenyl)-4,10-dihydropyrazolo[5,1 -c][1,4]benzoxazepin-7-yl]-2-methyl-propanamide,
2-methyl-N-[3-methyl-2-(o-tolyl)-4,10-dihydropyrazolo[5,1-c][1,4]benzoxazepin-7-yl]propanamide,
N-[3-methyl-2-(o-tolyl)-4,10-dihydropyrazolo[5,1-c][1,4]benzoxazepin-7-yl]acetamide,
N-[3-bromo-2-(2-methoxyphenyl)-4,10-dihydropyrazolo[5,1-c][1,4]benzoxazepin-7-yl]cyclopropanecarboxam ide,
N-[3-bromo-2-(o-tolyl)-4,10-dihydropyrazolo[5,1-c][1,4]benzoxazepin-7-yl]-2-methyl-propanam ide,
N-[3-fluoro-2-(o-tolyl)-4,10-dihydropyrazolo[5,1-c][1,4]benzoxazepin-7-yl]cyclopropanecarboxam ide,
N-[3-bromo-2-(o-tolyl)-4,10-dihydropyrazolo[5,1-c][1,4]benzoxazepin-7-yl]cyclopropanecarboxam ide,
N-[3-bromo-2-(o-tolyl)-4,10-dihydropyrazolo[5,1-c][1,4]benzoxazepin-7-yl]acetamide,
N-[3-bromo-2-(2-methoxyphenyl)-4,10-dihydropyrazolo[5,1-c][1,4]benzoxazepin-7-yl]acetamide,
N-[3-fluoro-2-(o-tolyl)-4,10-dihydropyrazolo[5,1-c][1,4]benzoxazepin-7-yl]acetamide, 2-methyl-N-[2-(o-tolyl)-4,10-dihydropyrazolo[5,1-c][1,4]benzoxazepin-7-yl]propanamide,
N-[2-(2-methoxyphenyl)-3-methyl-4,10-dihydropyrazolo[5,1-c][1,4]benzoxazepin-7-yl]acetamide,
N-[3,8-dichloro-2-(o-tolyl)-4,10-dihydropyrazolo[5,1-c][1,4]benzoxazepin-7-yl]-2-methyl-propanamide,
N-(6-fluoro-2-(o-tolyl)-4H,10H-benzo[f]pyrazolo[5,1-c][1,4]oxazepin-7-yl)propionamide
N-(2-(2-(trifluoromethyl)phenyl)-4H,10H-benzo[f]pyrazolo[5,1-c][1,4]oxazepin-7-yl)propionamide,
N-(2-(2-(trifluoromethyl)phenyl)-4H,10H-benzo[f]pyrazolo[5,1-c][1,4]oxazepin-7-yl)cyclopropanecarboxamide,
N-(2-(2,4-bis(trifluoromethyl)phenyl)-4H,10H-benzo[f]pyrazolo[5,1-c][1,4]oxazepin-7-yl)cyclopropanecarboxamide,
N-(2-(2-(trifluoromethyl)phenyl)-4H,10H-benzo[f]pyrazolo[5,1-c][1,4]oxazepin-7-yl)isobutyramide,
N-(2-(2,4-dimethylphenyl)-4H,10H-benzo[f]pyrazolo[5,1-c][1,4]oxazepin-7-yl)isobutyramide,
N-(2-(2-(trifluoromethyl)phenyl)-4H,10H-benzo[f]pyrazolo[5,1-c][1,4]oxazepin-7-yl)acetamide,
N-(2-(4-fluoro-2-methylphenyl)-4H,10H-benzo[f]pyrazolo[5,1-c][1,4]oxazepin-7-yl)propionamide,
N-(2-(5-fluoro-2-methylphenyl)-4H,10H-benzo[f]pyrazolo[5,1-c][1,4]oxazepin-7-yl)cyclopropanecarboxamide,
N-(2-(2-(trifluoromethoxy)phenyl)-4H,10H-benzo[f]pyrazolo[5,1-c][1,4]oxazepin-7-yl)propionamide,
N-(2-(2-(trifluoromethoxy)phenyl)-4H,10H-benzo[f]pyrazolo[5,1-c][1,4]oxazepin-7-yl)cyclopropanecarboxamide,
N-(2-(2-(trifluoromethoxy)phenyl)-4H,10H-benzo[f]pyrazolo[5,1-c][1,4]oxazepin-7-yl)isobutyramide,
N-(2-(2-(trifluoromethoxy)phenyl)-4H,10H-benzo[f]pyrazolo[5,1-c][1,4]oxazepin-7-yl)acetamide
N-(2-(o-tolyl)-4H,10H-benzo[f]pyrazolo[5,1-c][1,4]oxazepin-7-yl)cyclopropanecarboxamide.

### Chemical synthesis

The compounds of the present invention can be prepared following procedures well known to the person skilled in the art. For example, the compounds of general formula **(I)** can be disconnected to amine-intermediate **(X)** and acyl chloride, anhydride, or *in situ* activated carboxylic acid **(III)** (see **Scheme 1).** Amine intermediate **(X)** can be accessed *via* a palladium-catalysed cross coupling (e.g. Suzuki coupling) between organometallic compound, e.g. boronic acid **VI** and bromide **VII**. Bromide **VII** can be obtained via a seven-step synthetic procedure involving N-alkylation, reduction and cyclisation starting from commercially available 3-substituted pyrazole-5-carboxylate **(IV)** and commercially available 1-(bromomethyl)-2-methoxy-4-nitrobenzene **V.**

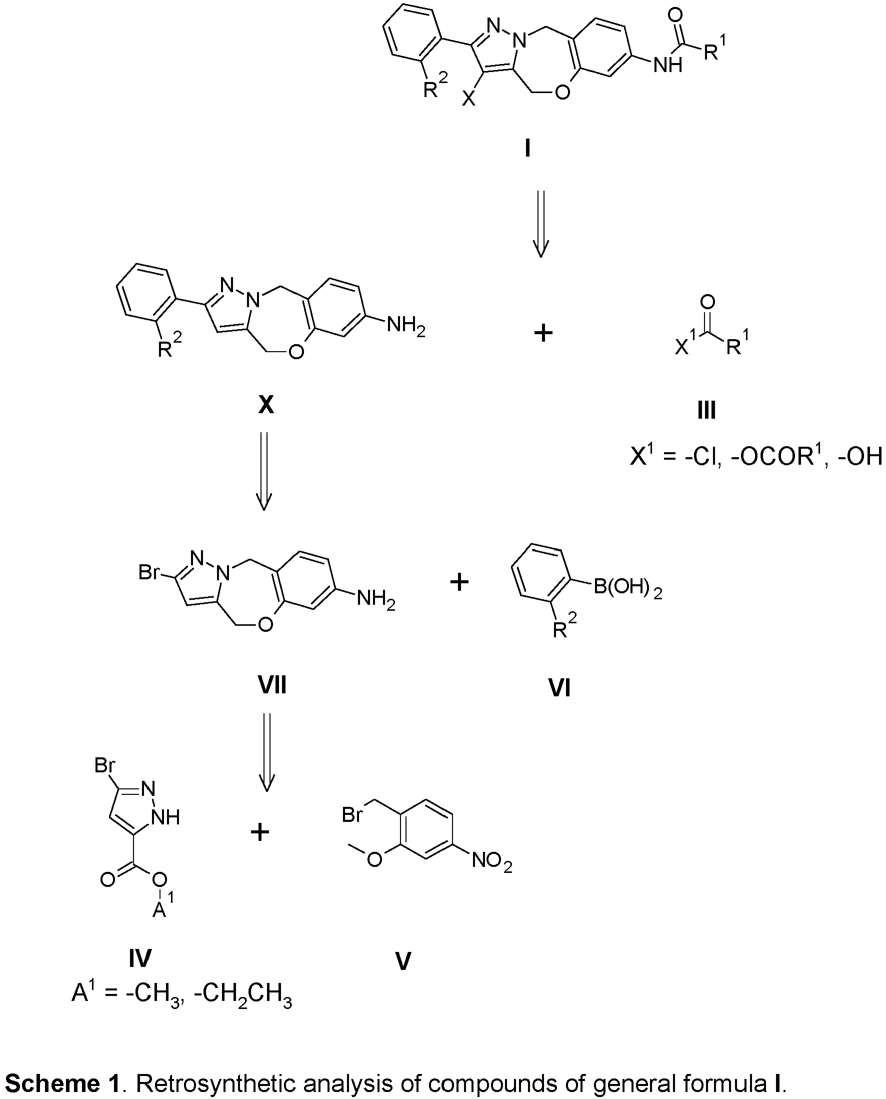

Intermediate **(VII)** may be obtained from boc-protected amine-intermediate **(VIII),** which can be prepared through a 7-step sequence from commercially available 3-substituted pyrazole-5-carboxylate **(IV)** and commercially available 1-(bromomethyl)-2-methoxy-4-nitrobenzene **V** as shown in **Scheme 2.** The synthesis commences with the alkylation of 3-substituted pyrazole-5-carboxylate **(IV)** with 1-(bromomethyl)-2-methoxy-4-nitrobenzene **V** in the presence of a strong base, such as sodium hydride or potassium carbonate. 2-Methoxy-4-nitrobenzyl pyrazole **IXa** was further converted to the corresponding 2-hydroxy-4-nitrobenzyl pyrazole **IXb** by demethylation using boron tribromide or other suitable Lewis acids such as aluminium chloride or aluminium bromide.

A person skilled in the art may envision that the synthesis of intermediate **VIII** as shown in **Scheme 2** may also be carried out starting from 1-(bromomethyl)-2-ethoxy-4-nitrobenzene **Va** instead of compound **V** and that conversion to the corresponding 2-hydroxy-4-nitrobenzyl pyrazole **IXb** can be performed under similar reaction conditions.

The ester **IXb** is then hydrolyzed to carboxylic acid **IXc** in the presence of a base in aqueous alcohol solution. Suitable bases are alkali metal hydroxides such as sodium hydroxide, potassium hydroxide or lithium hydroxide. The carboxylic acid **IXc** is then reduced to the corresponding primary alcohol **IXd** using a reducing agent such as borane dimethylsulfide or lithium aluminiumhydride. In a subsequent reduction step the nitro group of primary alcohol **IXd** is reduced to an amino group using iron and hydrochloric acid and protected with di-tert-butyl dicarbonate. Cyclisation of phenol **IXf** results in intermediate **V.**

A skilled person is aware that any other protecting group for amino groups common in the art is suitable for the protection of the amino group of compound **IXe,** such as acetate, carbonyloxybenzyl (Cbz), and fluorenylmethyloxycarbonyl (Fmoc).

An alternative synthetic route to access intermediate **VIII** is displayed in **Scheme 3.** Starting from **IV** and 1-(bromomethyl)-2-fluoro-4-nitrobenzene **Vb** instead of compound **V** compound **IXa'** is accessed as alkylation product. Subsequent hydrolysis leads to carboxylic acid **IXc',** which is then reduced to primary alcohol **IXd'** and amine **IXe'** and afterwards converted to N-Boc protected compound **IXf.** Cyclisation of fluoro-derivative **IXf'** in the presence of sodium hydride in polar DMF or DMSO results in intermediate **VIII.**

The synthetic access to intermediate **VIII** is not restricted to the syntheses described in **Schemes 2** and **3.** Intermediate **VIII** can be accessed by any suitable alkaline cyclisation method known in the art, e.g. Bunce, Richard A. et al., Journal of Heterocyclic Chemistry (2008), 45(2), 551-557 or Newman, Melvin S. et al., Journal of Organic Chemistry (1961), 26, 336-338.

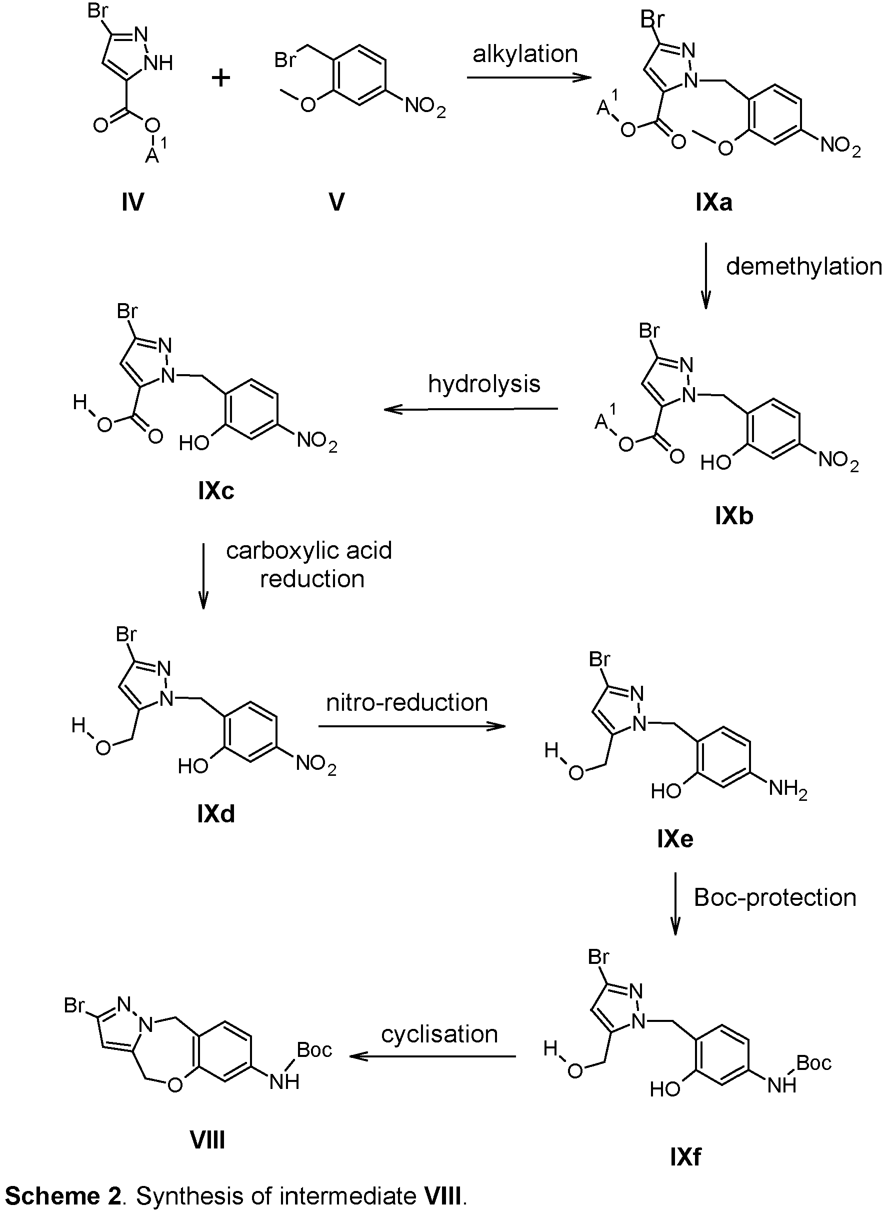

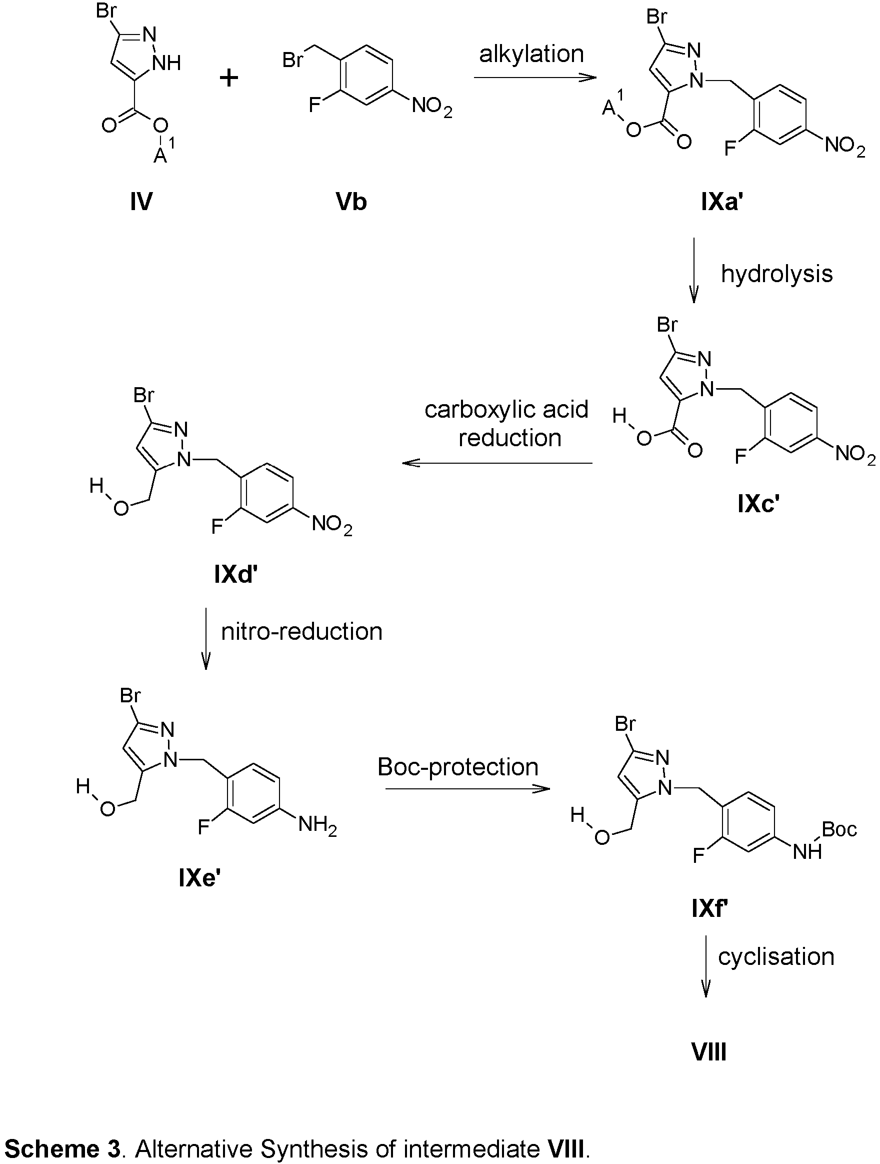

With intermediate **VIII** in hands the synthesis of the inventive compound of general formula **I** was accomplished using one of the synthetic pathways illustrated below (see **Schemes 4** to **6**).

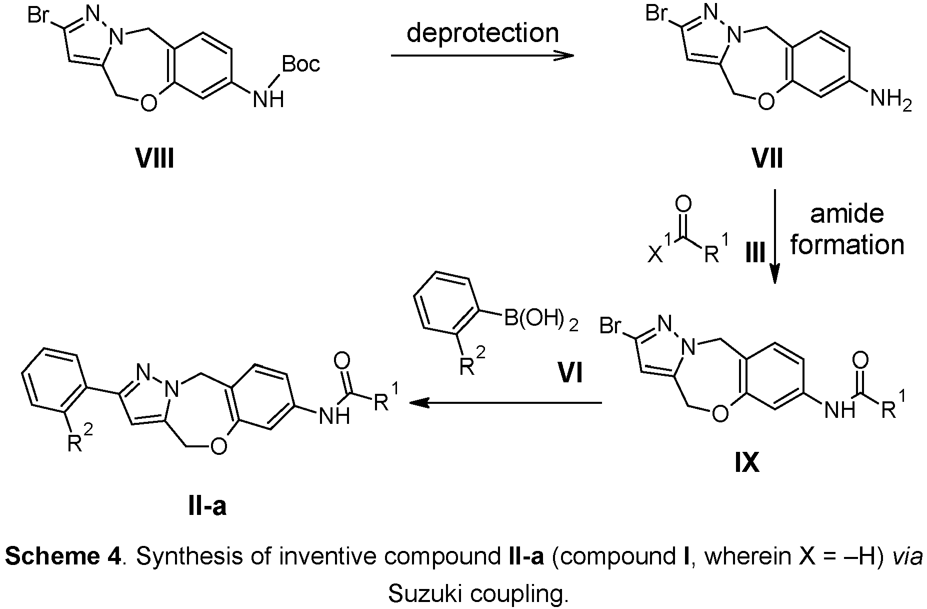

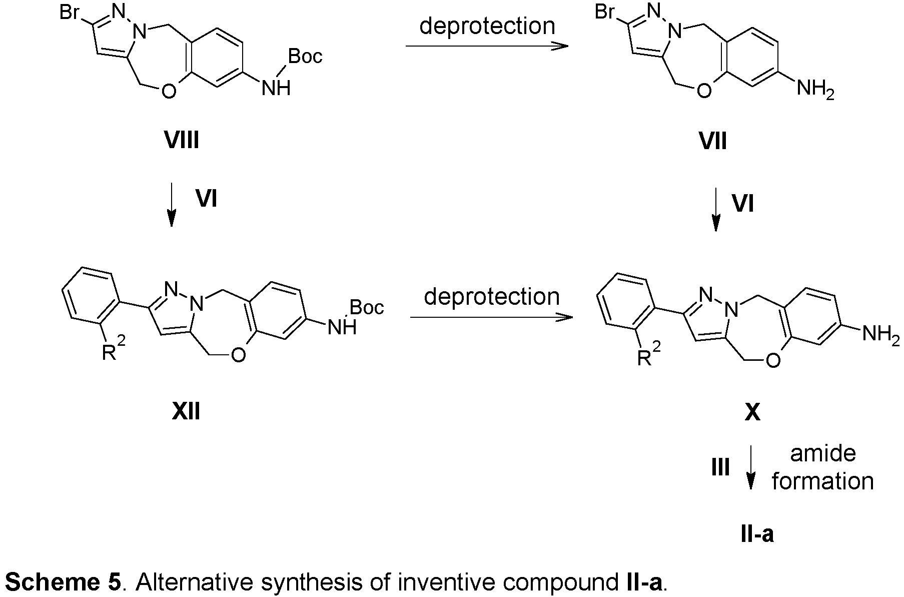

As illustrated in **Scheme 4,** the intermediate of general formula **VIII** can be converted to the free amine **VII** by treating with a strong acid, such as trifluoroacetic acid, hydrochloric acid or aqueous phosphoric acid. Access to amide **IX** is provided by reacting free amine **VII** with the corresponding acyl chloride, anhydride or *in situ* activated carboxylic acid **III.** Any *in situ* activation method described in the art (see for example Montalbetti et al. Tetrahedron 2005, 61, 10827-10852) is suitable for carboxylic acid **III.** Subjecting bromopyrazole **IX** to a palladium-catalyzed cross-coupling reaction with boronic acid **VI** gives access to compound **II-a**.

For a skilled person it is apparent that the synthetic steps outlined in **Scheme 4** can be reverted (see **Scheme 5**)**.** Hence, Boc-protected intermediate **VIII** is first reacted with boronic acid **VI** and subsequently deprotected and converted to amide **II-a** by reacting with compound **III.** Alternatively, Boc-protected intermediate **VIII** is deprotected first by treatment with trifluoroacetic acid and subsequently reacted with boronic acid **VI** before coupling with compound **III** to amide **II-a**.

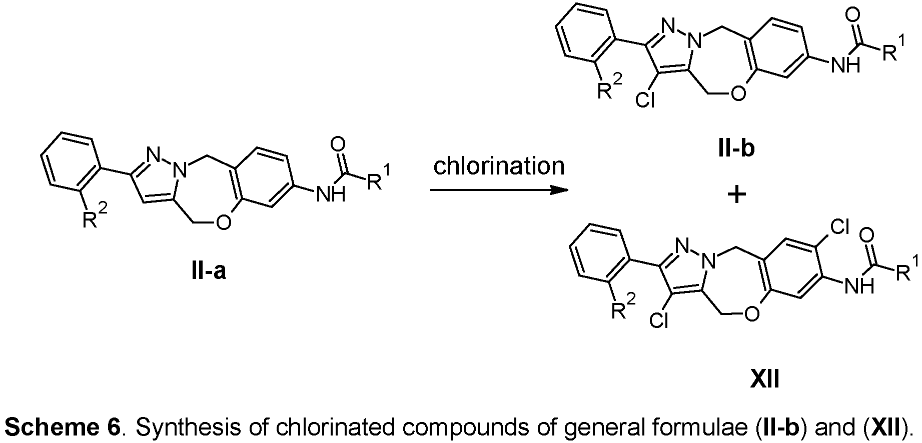

As illustrated in **Scheme 6,** pyrazolobenzoxazepine **II-a** can be further chlorinated using **N**-chlorosuccinimide, resulting in 3-chloro compounds **II-b** and 3,8-dichloro compounds **XII.**

The compounds of the present invention may form salts with organic or inorganic acids. Examples of suitable acids for such acid addition salt formation are trifluoroacetic acid, hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Some of the compounds of the present invention may be crystallized or recrystallized from solvents such as aqueous and organic solvents. In such cases solvates may be formed. This invention includes within its scope stoichiometric solvates including hydrates as well as compounds containing variable amounts of water that may be produced by processes such as lyophilization.

### Indications

Another aspect of the present invention relates to the use of the inventive compounds of general formulae **(I), (II-a)**, and **(II-b)** as drugs, i.e. as pharmaceutically active agents applicable in medicine.

### NET formation

As shown by IC₅₀ values determined by Sytox-Green staining assay for NET formation inhibitory activity, the compounds of general formulae **(I), (II-a)**, and **(II-b)** show a high potency (demonstrated by a low IC₅₀ value) for inhibiting neutrophil extracellular traps (NET) formation. Surprisingly, it turned out that the compounds according to the general formulae **(I), (II-a)**, and **(II-b)** as well as pharmaceutically acceptable salts thereof efficiently inhibit the formation of neutrophil extracellular traps from neutrophils after treatment with phorbol-12-myristate-13-acetate (PMA). Thus, the compounds according to general formulae **(I), (II-a)**, and **(II-b)**, as well as pharmaceutically acceptable salts thereof are useful as NET formation inhibitors.

As used herein the term "NET formation inhibitor" refers to a compound that downregulates, decreases and/or suppresses the formation of neutrophil extracellular traps.

Therefore, the present invention further relates to the use of compounds according to general formulae **(I), (II-a)**, and **(II-b)**, as well as pharmaceutically acceptable salts thereof for the prophylaxis and/or treatment of diseases associated with an enhanced NET formation and/or with an impaired NET degradation. In one embodiment of the present invention compounds according to general formulae **(I), (II-a)**, and **(II-b)**, as well as pharmaceutically acceptable salts thereof can be used for the prophylaxis and/or treatment of diseases associated with an enhanced NET formation and/or with an impaired NET degradation, wherein said diseases are selected from lupus erythematosus (SLE), lupus nephritis, hypocomplementemia, ANCA-associated vasculitis, antiphospholipid antibody syndrome, drug-induced vasculitis, atherosclerosis, rheumatoid arthritis, small vessel vascularitis, thrombosis, sepsis, (transfusion-related) acute lung injury, chronic obstructive pulmonary disease, cystic fibrosis, cancer metastases, pre-eclampsia, cardiac reperfusion, brain reperfusion, asthma, diabetes, diabetic wound healing, periodontitis, psoriasis, dermatomysitis, Polymyositis, malaria, endotoxemia, keratitis, dry-eye disease, Crohn's disease, irritable bowel disease, psoriasis, Alzheimer's disease, acute myocardial infarction, gout, Behcet's disease, infectious diseases caused by bacteria, virus, parasites or fungi, cancer, severe COVID-19, and Long COVID.

### Gasdermin D inhibition

Moreover, IC₅₀ values determined by LDH-Glo^{™} Assay after gasdermin D transfection, the compounds of general formulae **(I), (II), (II-A), (II-B), (II-C), (II-D), (II-E),** and **(II-F)** show a high potency (demonstrated by a low IC₅₀ value) for inhibiting Gasdermin-D activity. Surprisingly, it turned out that the compounds according to the general formulae **(I), (II), (II-A), (II-B), (II-C), (II-D), (II-E),** and **(II-F)** as well as pharmaceutically acceptable salts thereof efficiently inhibit gasdermin activitity, which causes and/or is associated with pyroptosis. Thus, the compounds according to general formulae **(I), (II), (II-A), (II-B), (II-C), (II-D), (II-E),** and **(II-F),** as well as pharmaceutically acceptable salts thereof are useful as GSDMD inhibitors.

Therefore, the present invention relates to compounds according to general formulae **(I), (II), (II-A), (II-B), (II-C), (II-D), (II-E),** and **(II-F),** as well as pharmaceutically acceptable salts thereof which are useful as GSDMD inhibitors and NET formation inhibitors.

Without being bound by theory, gasdermin D (GSDMD) inhibition is intrinsically associated with the prevention of pyroptosis. The term "pyroptosis" refers to a lytic form of programmed cell death characterized by cell swelling and release of cellular contents through lysis. Pyroptosis features rapid plasma-membrane rupture and release of proinflammatory intracellular contents. The characteristic membrane rupture is caused by GSDMD pores. Pyroptosis also results in the release of damage-associated molecular patterns (DAMPs) and inflammatory cytokines like IL-1β and IL-18, which are processed by caspase-1. GSDMD inhibition halts pore formation, preventing cytokine secretion and dampening inflammation. Pyroptosis further leads to the release of intracellular LDH into the extracellular space, a widely used marker of cell lysis. Upon cleavage by activated inflammatory caspases (e.g., caspase-1, caspase-4/5/11), the N-terminal fragment of GSDMD forms pores in the plasma membrane. These pores disrupt cellular homeostasis, leading to osmotic swelling, membrane rupture, and cell death.

GSDMD is thus the indispensable effector of pyroptosis, directly mediating the pore formation and membrane rupture that define this form of cell death. Inhibition of GSDMD specifically prevents pyroptosis without significantly affecting other forms of cell death, such as apoptosis or necroptosis. This association is strongly supported by genetic, biochemical, and pharmacological evidence, making GSDMD inhibition a compelling therapeutic strategy for inflammatory diseases.

Thus, the compounds according to general formulae **(I), (II), (II-A), (II-B), (II-C), (II-D), (II-E),** and **(II-F),** as well as pharmaceutically acceptable salts thereof are useful as pyroptosis inhibitors.

As used herein the term "GSDMD inhibitor" refers to a compound that downregulates, decreases and/or suppresses the biological activity of Gasdermin-D in the individual. Preferably, Gasdermin-D activity in the individual is reduced by at least about 10%, at least about 20%, at least about 30%, at least about 40%, at least about 50%, at least about 60%, at least about 70%, at least about 80%, at least about 90% or more relative to the level of Gasdermin-D activity in the individual prior to the treatment with the inventive compound of general formulae **(I), (II), (II-A), (II-B), (II-C), (II-D), (II-E),** and **(II-F).** Preferably, Gasdermin-D activity in the individual having an elevated level of Gasdermin-D activity is reduced to a level of Gasdermin-D activity that is present in a healthy individual.

As used herein the term "pyroptosis inhibitor" refers to a compound that downregulates, decreases and/or suppresses the occurrence of pyroptosis in the individual. Preferably, pyroptosis in the individual experiencing pyroptosis is reduced by at least about 10%, at least about 20%, at least about 30%, at least about 40%, at least about 50%, at least about 60%, at least about 70%, at least about 80%, at least about 90% or more relative to the level of pyroptosis in the individual prior to the treatment with the inventive compound of general formulae **(I), (II), (II-A), (II-B), (II-C), (II-D), (II-E),** and **(II-F).** Preferably, pyroptosis in the individual having an elevated level of pyroptosis is reduced to a level of pyroptosis that is present in a healthy individual.

Without being bound by theory, the inventive compounds of general formulae **(I), (II)**, **(II-A), (II-B), (II-C), (II-D), (II-E),** and **(II-F),** as well as pharmaceutically acceptable salts thereof are thought to act as a dual inhibitor of NET formation and pyroptosis are thus useful for the prophylaxis and/or treatment of a broad-spectrum of inflammatory diseases, where both pathways contribute significantly to disease progression. Such inflammatory diseases often involve hyperactivation of both pyroptosis and NET formation. A dual inhibitor dampens systemic inflammation while preserving other immune functions. The inventive compounds of general formulae **(I), (II)**, **(II-A), (II-B), (II-C), (II-D), (II-E),** and **(II-F),** as well as pharmaceutically acceptable salts thereof are thought to inhibit the activity of inflammasomes that otherwise would induce the production of cytokines IL-1β and IL-18 and gasdermin D pore formation by caspase activation, without inhibiting the maturation and release of cytokines IL-1β and IL-18 in the presence of the inventive compounds. Therefore, the present invention further relates to the use of compounds according to general formulae **((I), (II)**, **(II-A), (II-B), (II-C), (II-D), (II-E),** and **(II-F),** as well as pharmaceutically acceptable salts thereof for the prophylaxis and/or treatment of diseases and conditions associated with and/or caused by an overactivity of inflammasomes.

Several studies have shown the critical role of gasdermin-mediated pyroptosis in various diseases, including **infectious diseases** (e.g., sepsis, viral infections), **inflammatory diseases** (e.g., IBD, pancreatitis, scleroderma, rheumatoid arthritis, systemic lupus erythematosus), **neurodegenerative diseases** (e.g., multiple sclerosis, stroke, Alzheimer's, Parkinson's), **metabolic disorders** (e.g., type 2 diabetes, thrombosis, long COVID, NASH), **cardiovascular diseases** (e.g., atherosclerosis, myocardial infarction), **cancer** (e.g., gastric and colorectal), and **toxin-related conditions** (e.g., environmental toxin toxicity).

Regarding sepsis, gasdermin D (GSDMD)-mediated pyroptosis plays a pivotal role in excessive inflammation and systemic organ dysfunction in sepsis. Studies demonstrate that GSDMD knockout (KO) reduces sepsis-induced mortality and alleviates associated side effects. Similarly, the GSDMD inhibitor disulfiram effectively mitigates sepsis-induced lethality. Regarding viral Infections, GSDMD-dependent pyroptosis contributes to pathology in viral infections. For instance, GSDMD KO mice exhibit significantly attenuated weight loss, lung dysfunction, histopathology, and mortality compared to wild-type (WT) mice following viral challenge.

Regarding inflammatory diseases, dysregulation of GSDMD-mediated pyroptosis exacerbates intestinal inflammation. In murine colitis models (e.g., dextran sulfate sodium-induced colitis), GSDMD KO mice demonstrate reduced inflammation and tissue damage relative to WT controls. Genetic studies also implicate single nucleotide polymorphisms (SNPs) in GSDMA, GSDMB, GSDMD, and GSDME genes in IBD susceptibility.

Regarding neurodegenerative diseases, pyroptosis contributes to neuroinflammation and demyelination in progressive MS. GSDMD activation in oligodendrocytes and microglia drives disease progression, while GSDMD KO reduces inflammation and preserves neurological performance. Further studies have shown that inhibition of GSDMD reduces neutrophil extracellular trap (NET) formation, lesion size, and other biomarkers in ischemic stroke and subarachnoid hemorrhage models.

Regarding Long COVID, NETs, mediated by GSDMD, play a key role in thrombus formation in venous, arterial, and cancer-associated thrombosis. NET formation is also linked to COVID-19-induced microthrombi and ARDS pathology. Inhibitors and GSDMD KO prevent NET-mediated thrombotic angiopathy, a hallmark of long COVID.

Regarding Type 2 diabetes, pyroptosis contributes to beta-cell death and insulin resistance. GSDMD KO alleviates diabetic nephropathy and improves podocyte function, while disulfiram accelerates wound healing in a GSDMD-dependent manner.

Regarding cardiovasular diseases, GSDMD-mediated pyroptosis contributes to inflammation, plaque instability, and cardiomyocyte death following MI. GSDMD KO and inhibitors offer cardioprotection.

Regarding cancer, GSDMD dysregulation is implicated in the development and progression of gastric and colorectal cancers. GSDMD inhibition may also reduce chemotherapy-related side effects.

Hence, the compounds of general formulae **(I), (II)**, **(II-A), (II-B), (II-C), (II-D), (II-E),** and **(II-F),** pharmaceutically acceptable salts thereof, as well as the pharmaceutical compositions comprising a compound of general formula **(I)** or a pharmaceutically acceptable salt thereof are useful for treatment and/or prophylaxis of proliferative diseases, tumours, cancers, metastases, immunological disorders, autoimmune diseases and inflammatory disorders.

Thus, the compounds of the present invention can be used for prophylaxis and/or treatment of proliferative diseases, tumours, cancers, metastases, immunological disorders, infectious diseases, autoimmune diseases and inflammatory disorders or for the preparation of a pharmaceutical formulation for prophylaxis and treatment of proliferative diseases, tumours, cancers, metastases, immunological disorders, infectious diseases, autoimmune diseases and inflammatory disorders.

More specifically, proliferative diseases, tumours, cancers or metastases that can be treated and/or prevented by the inventive compounds are selected from the group comprising or consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, estrogen dependent and independent breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's lymphomas), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma, tongue cancer, invasive ductal carcinoma, invasive lobular carcinoma, ductal carcinoma in situ, lobular carcinoma in situ, small-cell lung carcinoma, non-small-cell lung carcinoma, bronchial adenoma, pleuropulmonary blastoma, mesothelioma, brain stem glioma, hypophtalmic glioma, cerebellar astrocytoma, cerebral astrocytoma, neuroectodermal tumours, pineal tumors, sarcoma of the uterus, salivary gland cancers, anal gland adenocarcinomas, mast cell tumors, pelvis tumours, ureter tumours, hereditary papillary renal cancers, sporadic papillary renal cancers, intraocular melanoma, hepatocellular carcinoma (liver cell carcinomas with or without fibrolamellar variant), cholangiocarcinoma (intrahepatic bile duct carcinoma), mixed hepatocellular cholangiocarcinoma, squamous cell carcinoma, malignant melanoma, Merkel cell skin cancer, non-melanoma skin cancer, hypopharyngeal cancer, nasopharyngeal cancer, oropharyngeal cancer, oral cavity cancer, squamous cell cancer, oral melanoma, AIDS-related lymphoma, cutaneous T-cell lymphoma, lymphoma of the central nervous system, malignant fibrous histiocytoma, lymphosarcoma, rhabdomyosarcoma, malignant histiocytosis, fibrosarcoma, hemangiosarcoma, hemangiopericytoma, leiomyosarcoma., canine mammary carcinoma, and feline mammary carcinoma.

Furthermore, the compounds of general **(I), (II)**, **(II-A), (II-B), (II-C), (II-D), (II-E),** and **(II-F)** are useful for the treatment and/or prophylaxis of immunological disorders and/or autoimmune diseases, wherein the immunological disorders and/or autoimmune diseases are selected from the group comprising or consisting of: asthma, diabetes, rheumatic diseases, AIDS, rejection of transplanted organs and tissues, rhinitis, chronic obstructive pulmonary diseases, osteoporosis, ulcerative colitis, sinusitis, lupus erythematosus, recurrent infections, atopic dermatitis / eczema and occupational allergies, food allergies, drug allergies, severe anaphylactic reactions, anaphylaxis, manifestations of allergic diseases, primary immunodeficiencies, antibody deficiency states, cell mediated immunodeficiencies, severe combined immunodeficiency, DiGeorge syndrome, Hyper-lgE syndrome, Wiskott-Aldrich syndrome, ataxia-telangiectasia, immune mediated cancers, white cell defects, autoimmune diseases, systemic lupus erythematosus, rheumatoid arthritis (RA), multiple sclerosis (MS), immune-mediated or Type 1 Diabetes Mellitus, immune mediated glomerulonephritis, scleroderma, pernicious anemia, alopecia, pemphigus, pemphigus vulgaris, myasthenia gravis, inflammatory bowel diseases, Crohn's disease, psoriasis, autoimmune thyroid diseases, Hashimoto's disease, dermatomyositis, goodpastture syndrome, myasthenia gravis pseudoparalytica, ophtalmia sympatica, phakogene uveitis, chronical agressive hepatitis, primary billiary cirrhosis, autoimunehemolytic anemy, and Werlof disease.

Also, the compounds of general formulae **(I), (II), (II-A), (II-B), (II-C), (II-D), (II-E),** and **(II-F)** are useful for the treatment and/or prophylaxis of infectious diseases, wherein the infectious diseases are selected from the group of bacterial, parasitic, fungal or viral infectious diseases.

The compounds of general formulae **(I), (II), (II-A), (II-B), (II-C), (II-D), (II-E),** and **(II-F)** are useful for the treatment and/or prophylaxis of bacterial infectious diseases caused by a pathogen selected from the group comprising:
Allochromatium vinosum, Acinetobacter baumanii, Bacillus anthracis, Campylobacter jejuni, Clostridium spp., Citrobacter spp., Escherichia coli, Enterobacter spp., Enterococcus faecalis., Enterococcus faecium, Francisella tularensis, Haemophilus influenzae, Helicobacter pylori, Klebsiella spp., Listeria monocytogenes, Moraxella catharralis, Mycobacterium tuberculosis, Neisseria meningitidis, Neisseria gonorrhoeae, Proteus mirabilis, Proteus vulgaris, Pseudomonas aeruginosa, Salmonella spp., Serratia spp., Shigella spp., Stenotrophomonas maltophilia, Staphylococcus aureus, Staphylococcus epidermidis, Streptococcus pneumoniae, Streptococcus pyogenes, Streptococcus agalactiae, Yersina pestis, and Yersina enterocolitica.

The compounds of general formulae **(I), (II), (II-A), (II-B), (II-C), (II-D), (II-E),** and **(II-F)** are useful for the treatment and/or prophylaxis of parasitic infectious diseases caused by a pathogen selected from the group comprising:
Babesia, Balantidium, Besnoitia, Blastocystis, Coccidia, Cryptosporidium, Cytauxzoon, Cyclospora, Dientamoeba, Eimeria, Entamoeba, Enterocytozoon, Enzephalitozoon, Eperythrozoon, Giardia, Hammondia, Isospora, Leishmania, Microsporidia, Naegleria, Plasmodium, Plasmodium falciparum, Plasmodium vivax, Plasmodium ovale, Plasmodium malariae, Plasmodium knowlesi, Pneumocystis, Schistosoma, Sarcocystis, Theileria, Trichinella, Toxoplasma, Trichomonas, Trypanosoma, Unicaria, Cestoda, Dipylidium, Dranunculus, Echinococcus, Fasciola, Fasciolopsis, Taenia, Ancylostoma, Ascaris, Brugia, Enterobius, Loa loa, Mansonella, Necator, Oncocerca, Strongyloides, Strongylus, Toxocara, Toxascaris, Trichuris, and Wucheria.

The compounds of general formulae **(I), (II)**, **(II-A), (II-B), (II-C), (II-D), (II-E),** and **(II-F)** are useful for the treatment and/or prophylaxis of fungal infectious diseases caused by a pathogen selected from the group comprising:
Trichophyton mentagrophytes, Trichophyton rubrum, Trichophyton interdigitale, T. schönleinii, T. verrucosum, T. violaceum, T. tonsurans, Trichophyton spp., M. canis, Candida albicans, C. guillermondii, C. krusei, C. parapsilosis, C. tropicalis, C. glabrata, Candida spp., Microsporum spp., Microsporum canis, Microsporum audonii, Microsporum gypseum, M. ferrugineum, Trichosporum beigelii, Trichosporum inkiin, Aspergillus niger, Alternaria, Acremonium, Fusarium, and Scopulariopsis.

The compounds of general formulae **(I), (II), (II-A), (II-B), (II-C), (II-D), (II-E),** and **(II-F)** are useful for the treatment and/or prophylaxis of viral infectious diseases caused by a pathogen selected from the group comprising:
Adenoviruses, Ebolavirus, Epstein-Barr-virus, Flavivirus, FSME-virus, Influenza virus, Hanta-virus, human immunodeficiency virus ("HIV"), herpes simplex virus ("HSV", type 1 or 2), human herpes virus 6 (HHV-6), human Papilloma virus ("HPV", type 16 or 18), human Cytomegalovirus ("HCMV"), human hepatitis B or C virus ("HBV", Type B; "HCV", type C), Lassavirus, Lyssavirus (EBL 1 or EBL 2), Marburgvirus, Norovirus, Parvovirus B19, Pestivirus, Poliovirus, respiratory syncytial virus, Rhinovirus, Rotaviruses, SARS-associated Coronavirus, Varicella-Zoster virus, coronavirus, and SARS-CoV-2 virus.

Additionally, the inventive compounds of general formulae **(I), (II), (II-A), (II-B), (II-C), (II-D), (II-E),** and **(II-F)** are useful for the treatment and/or prophylaxis of inflammatory disorders, wherein the inflammatory disorders are selected from the group comprising or consisting of: abscessation, acanthameba, acanthamebiasis, acne vulgaris, actinomycosis, acute inflammatory dermatoses, acute laryngeal infections of adults, acute multifocal placoid pigmentary epitheliopathy, acute (thermal) injury, acute retinal necrosis, acute suppurative otitis media, algal disorders, allergic contact dermatitis, amyloidosis angioedema, ankylosing spondylitis, aspergillosis, atopic dermatitis, Aujeszky's disease, autoantibodies in vasculitis, babesiosis, bacterial disorders, bacterial laryngitis, bacterial meningitis, Behcet's disease, birdshot choroidopathy, blastomycosis, borna disease, brucellosis, bullous myringitis, bursitis, candidiasis, canine distemper encephalomyelitis, canine distemper encephalomyelitis in immature animals, canine ehrlichiosis, canine herpes virus encephalomyelitis, cholesteatoma, chronic (granulomatous) diseases, chronic inflammatory dermatoses, chronic relapsing encephalomyelitis, chronic suppurative otitis media, cicatricial pemphigoid, coccidiomycosis, coccidioidomycosis, common upper respiratory infection, contact ulcer and granuloma, Crohn's disease, cryptococcosis, cysticercosis, dermatomyositis, diphtheria, discoid lupus erythematosus, drug-induced vasculitis, drug or hypersensitivity reaction, encephalitozoonosis, eosinophilic meningoencephalitis, erythemal multiforme (EM minor), feline leukemia virus, feline immunodeficiency virus, feline infectious peritonitis, feline polioencephalomyelitis, feline spongiform encephalopathy, fibromyositis, Fuch's heterochromic cyclitis, gastroesophageal (laryngopharyngeal) reflux disease, giant cell arteritis, glanders, glaucomatocyclitic crisis, gonorrhea granular myringitis, granulomatous meningoencephalomyelitis, herpes simplex, histoplasmosis, idiopathic diseases, idiopathic inflammatory disorders, immune and idiopathic disorders, infections of the immunocompromised host, infectious canine hepatitis, inhalation laryngitis, interstitial nephritis, irritant contact dermatitis, juvenile rheumatoid arthritis, Kawasaki's disease, La Crosse virus encephalitis, laryngeal abscess, laryngotracheitis (croup), leishmaniasis, lens-induced uveitis, leprosy, leptospirosis, leukemia, lichen planus, lupus, lyme disease, lymphoma, meningitis, meningoencephalitis in greyhounds, miscellaneous meningitis / meningoencephalitis, microscopic polyangiitis, multifocal choroiditis, multifocal distemper encephalomyelitis in mature animals, multiple sclerosis, muscle tension dysphonias, mycotic (fungal) diseases, mycotic diseases of the CNS, necrotizing encephalitis, neosporosis, old dog encephalitis, onchocerciasis, parasitic encephalomyelitis, parasitic infections, pars planitis, parvovirus encephalitis, pediatric laryngitis, pollution and inhalant allergy, polymyositis, post-vaccinal canine distemper encephalitis, post-vaccinal rabies, prion protein induced diseases, protothecosis, protozoal encephalitis-encephalomyelitis, psoriasis, psoriatic arthritis, pug dog encephalitis, pyogranulomatous meningoencephalomyelitis, rabies, radiation injury, radiation laryngitis, radionecrosis, relapsing polychondritis, Reiters's syndrome, retinitis pigmentosa, retinoblastoma, rheumatoid arthritis, rickettsial disorders, rocky mountain spotted fever, salmon poisoning, sarcocystosis, sarcoidosis, schistosomiasis, scleroderma, scleroma, serpiginous choroiditis, shaker dog disease, Sjogren's syndrome, spasmodic croup, spirochetal (syphilis) diseases, spongiotic dermatitis, sporotrichosis, steroid responsive meningitis-arteritis, Stevens-Johnson syndrome (SJS, EM major), supraglottitis (epiglottitis), sympathetic ophthalmia, syngamus laryngeus, syphilis, systemic lupus erythematosus, systemic vasculitis in sarcoidosis, Takayasu's arteritis, tendinitis (tendonitis), thromboangiitis obliterans (Buerger's Disease), tick-borne encephalitis in dogs, toxic epidermal necrolysis (TEN), toxocariasis, toxoplasmosis, trauma, traumatic laryngitis, trichinosis, trypanosomiasis, tuberculosis, tularemia, ulcerative colitis, urticaria (hives), vasculitis, vasculitis and malignancy, vasculitis and rheumatoid arthritis, vasculitis in systemic lupus erythematosus, vasculitis in the idiopathic inflammatory myopathies, vasculitis of the central nervous system, vasculitis secondary to bacterial, fungal, and parasitic infection, viral disorders, viral laryngitis, vitiligo, vocal abuse, vocal-cord hemorrhage, Vogt Koyanagi Harada syndrome, Wegener's granulomatosis, Whipple's disease, pancreatitis, severe COVID-19 and Long COVID.

Moreover, the inventive compounds of general formulae **(I), (II)**, **(II-A), (II-B), (II-C), (II-D), (II-E),** and **(II-F)** are useful for the treatment and/or prophylaxis of circulatory diseases, wherein the circulatory diseases are selected from the group comprising or consisting of: high blood pressure, atherosclerosis, coronary artery disease, heart attack, heart failure, stroke, abdominal aortic aneurism, peripheral artery disease, myocardial infarction, mitral prolapse, stenosis, regurgitation, angina pectoris, arrhythmia, dysrhythmia and ischemia.

### Pharmaceutical compositions

Another aspect of the present invention is directed to pharmaceutical compositions comprising at least one compound of the present invention as active agent, together with at least one pharmaceutically acceptable carrier, excipient and/or diluent. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention.

The pharmaceutical compositions according to the present invention containing at least one compound according to the present invention as active agent will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the inventive compound of general formulae **(I), (II), (II-A), (II-B), (II-C), (II-D), (II-E),** and **(II-F)** as active agent.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimize the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized molds, allowed to cool, and thereby solidified.

Also included are solid form preparations that are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives. Under tablet a compressed or molded solid dosage form is understood, which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilized in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances, which are added to the dosage form to enable the tablet granules etc. after being compressed to release from the mold or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

The pharmaceutical compositions of the present invention are suitable for the treatment and/or prophylaxis of proliferative diseases, tumours, cancers, metastases, immunological disorders, autoimmune diseases and inflammatory disorders.

Another aspect of the present invention relates to drug combinations and pharmaceutical compositions comprising at least one compound of general formulae **(I), (II), (II-A), (II-B), (II-C), (II-D), (II-E),** and **(II-F)** as active agent together with at least one pharmaceutically acceptable carrier, excipient and/or diluent and one or more other anti-tumor agents or anti-retroviral drugs. As used herein the term "drug combination" refers to a combination of at least two pharmaceutically active agents or therapeutic agents with or without further ingredients, carrier, diluents and/or solvents.

Compounds of formulae **(I), (II), (II-A), (II-B), (II-C), (II-D), (II-E),** and **(II-F)** may be administered as the sole pharmaceutical agent or in combination with one or more additional therapeutic agents, wherein the drug combination causes no unacceptable adverse effects. This combination therapy includes administration of a single pharmaceutical dosage formulation, which contains a compound of any one of formulae **(I), (II), (II-A), (II-B), (II-C), (II-D), (II-E),** and **(II-F)** and one or more additional therapeutic agents in form of a single pharmaceutical composition, as well as administration of the compound of formulae ((**I**), **(II)**, **(II-A), (II-B), (II-C), (II-D), (II-E),** and **(II-F)** and each additional therapeutic agent in its own separate pharmaceutical dosage formulation, i.e. in its own separate pharmaceutical composition. For example, a compound of any one of formula **(I), (II-a)**, and **(II-b)** and a therapeutic agent may be administered to the patient together in a single oral dosage composition such as a tablet or capsule, or each agent may be administered in separate pharmaceutical compositions.

Where separate pharmaceutical compositions are used, the compound of formulae **(I), (II), (II-A), (II-B), (II-C), (II-D), (II-E),** and **(II-F)** and one or more additional therapeutic agents may be administered at essentially the same time (e.g. concurrently) or at separately staggered times (e.g. sequentially).

The compounds of the present invention may also be employed in cancer treatment in conjunction with radiation therapy and/or surgical intervention.

Furthermore, the compounds of formulae **(I), (II-a)**, and **(II-b)** may be utilized, as such or in compositions, in research and diagnostics, or as analytical reference standards, and the like, which are well known in the art.

### Description of Figures

**Fig 1****.** shows the experimental time schedule.
**Fig 2****.** shows randomization before compound treatment.
**Fig 3****.** shows neurological deficits of animals in the MCAO group at 6 hours after ischemia.
**Fig 4****.** shows neurological deficits of animals in the MCAO group at 24 hours after ischemia.
**Fig 5****.** shows representative images of TTC staining at 6 h Post-Ischemia
**Fig 6****.** shows quantitative morphometry of TTC staining at 6 h Post-Ischemia
**Fig 7****.** shows representative images of TTC staining at 24 h Post-Ischemia
**Fig 8****.** shows quantitative morphometry of TTC staining at 24 h Post-Ischemia

The following examples are included to demonstrate preferred embodiments of the invention. It should be appreciated by those of skill in the art that the techniques disclosed in the following examples represent techniques discovered by the inventor to function well in the practice of the invention, and thus can be considered to constitute preferred modes for its practice. However, those of skill in the art should, in light of the present disclosure, appreciate that many changes can be made in the specific embodiments, which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### A. Chemical Synthesis

### Abbreviations and Acronyms:

- AAALAC: Association for Assessment and Accreditation of Laboratory Animal Care
- ANOVA: Analysis of variance
- aq.: aqueous
- BBrs: boron tribromide
- Boc: *tert*-Butoxycarbonyl
- br.: broad
- CAS: CAS Registry Number (CAS = Chemical Abstracts Service)
- CCA: Common Carotid Artery
- CHCl₃: chloroform
- cHex: cyclohexane
- d: doublet
- dd: doublet of doublets
- DCM: dichloromethane
- DEAD: diethyl azodicarboxylate
- DIPEA: N,N-diisopropyl-ethylamine
- DME: dimethoxyethane
- DMF: dimethylformamide
- DMSO: dimethylsulfoxide
- ECA: External Carotid Artery
- EDTA: 2,2',2",2‴-(Ethane-1,2-diyldinitrilo)tetraacetic acid
- ES: electrospray
- EtOAc: ethyl acetate
- FCS: fetal calf serum
- FFPE: Formalin Fixed Paraffin-Embedded
- GSDMD: Gasdermin-D
- h: hour
- HCl: hydrochloric acid
- HDB: Housing and Development Board Animals Rules
- HEK293T: embryonic kidney fibroblast cell line
- H₂O: water
- hPBMC: human Peripheral Blood Mononuclear Cells
- IC₅₀: half maximal inhibitory concentration
- ICA: Internal Carotid Artery
- K₂CO₃: potassium carbonate
- LDH: lactate dehydrogenase
- m: multiplet
- MCA: Middle Cerebral Artery
- MCAO: Middle Cerebral Artery Occlusion
- MeCN: acetonitrile
- MeOH: methanol
- MgSO₄: magnesium sulphate
- min: minutes
- MS: mass spectrometry
- NaHCOs: sodium hydrogencarbonate
- NaH: sodium hydride
- NaOH: sodium hydroxyde
- Na₂SO₄: sodium sulphate
- NCS: N-chlorosuccinimide
- NET: neutrophil extracellular traps
- NMR: nuclear magnetic resonance
- PBS: phosphate-buffered saline
- Pd(PPh₃)₄: Tetrakis(triphenylphosphine)palladium(0)
- PMA: phorbol 12-myristate 13-acetate
- PPhs: triphenylphosphine
- p: pentet
- q: quartet
- rt: room temperature
- s: singlet
- sat.: saturated
- SEM: standard error of the mean
- sep: septet
- t: triplet
- TFA: trifluoroacetic acid
- THF: tetrahydrofuran
- THP1: acute monocytic leukaemia cancer cell line
- TTC: 2,3,5-triphenyl tetrazolium chloride

### LC-MS method

HPLC - electrospray mass spectra (HPLC ES-MS) were obtained using a Waters Acquity Ultra Performance Liquid Chromatography (UPLC) equipped SQ 3100 Mass detector spectrometer.
Column: Acquity UPLC BEH C18 1.7 µm, 2.1x50 mm
Flow: 0.500 mL/min
Eluents: A: H₂O with 0.05% formic acid and B: MeCN with 0.05% formic acid.
Gradient: elution from 5% to 100% B over 3.5 min with an initial hold of 0.5 min and a final hold at 100% B of 0.5 min. Total run time: 5 min.

The gradient described could be altered in function of the physico-chemical properties of the compound analyzed and is in no way restrictive.

### Preparative HPLC method

Preparative HPLC was performed using a Waters System consisting of a Waters 2767 Sample Manager, a Waters 2545 Binary Gradient Module, a Waters SFO (System Fluidics Organizer), a Waters 3100 Mass Detector, and a Waters 2498 UV/Visible Detector.
Column: XBridge^{®} Prep C18 5 µm OBD^{™}, 19 x 150 mm
Flow: 20 mL/min
Eluents: A: H₂O with 0.1% TFA and B: MeCN with 0.1% TFA.
General Gradient: Elution from X% to Y% B over 13min with an initial hold of 2 min and a final increase to 100% B over 13 min and hold at 100% B of 2 min followed by a 0.5 min gradient back to the initial composition and hold at initial composition of 0.5 min. Total run time: 20 min. X = Y - 30% where Y = concentration of elution on the above described LC-MS method.

The gradient described could be altered in function of the physico-chemical properties of the compound analyzed and is in no way restrictive.

### NMR methods

Proton (¹H) nuclear magnetic resonance (NMR) spectra were measured with an Oxford Varian 400/54 (400MHz) spectrometer or a Bruker Avance II (300MHz) spectrometer with residual protonated solvent (CHCl₃ δ 7.26; MeOH δ 3.30; DMSO δ 2.49) as standard. The NMR data of the synthesized examples, some of which are not disclosed, are in agreement with their corresponding structural assignments.

### Example A.1: Synthesis of methyl 3-bromo-1-(2-methoxy-4-nitrobenzyl)-1H-pyrazole-5-carboxylate (1)

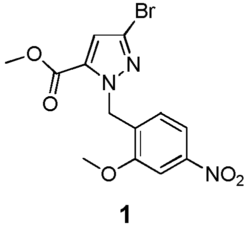

To a stirred solution of methyl 3-bromo-1H-pyrazole-5-carboxylate (64.7 g; 316 mmol) in DMF (900 mL) NaH (8.7 g; 363 mmol) was added portion-wise. The stirring was continued for 1 h and 1-(bromomethyl)-2-methoxy-4-nitrobenzene (77.6 g; 316 mmol) in a minimal amount of DMF was added dropwise. The mixture was stirred at 50 °C overnight, evaporated, diluted with water, and extracted with ethyl acetate. The combined organic layers were dried over Na₂SO₄, filtered and evaporated *in vacuo.* The residue was purified by column chromatography on silica gel to yield the desired product **1** (54 g; 46%) as a yellow solid.

¹H NMR (400 MHz, Chloroform-d) δ 7.71 (d, *J* = 2.1 Hz, 1H), 7.69 (s, 1H), 6.89 (s, 1H), 6.65 (d, *J* = 8.2 Hz, 1H), 5.77 (s, 2H), 3.94 (s, 3H), 3.81 (s, 3H).

### Example A.2: Synthesis of methyl 3-bromo-1-(2-hydroxy-4-nitrobenzyl)-1H-pyrazole-5-carboxylate (2)

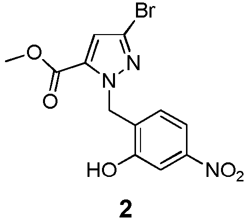

To a cooled to -78 °C and stirred mixture of **1** (54 g; 146 mmol) in dry DCM (700 mL) BBrs (110 g; 438 mmol) in DCM (150 mL) was added under argon atmosphere. The cooling bath was removed, and the mixture was slowly warmed to rt. The mixture was diluted with water and stirred for 30 min. The organic layer was separated, dried over Na₂SO₄, filtered and evaporated *in vacuo.* The residue was diluted with hexane and the precipitate was filtered, to yield the desired product **2** (50 g; 96%) as a yellow solid.

¹H NMR (400 MHz, DMSO-*d*₆) δ 10.90 (s, 1H), 7.66 - 7.59 (m, 2H), 7.13 (d, *J* = 1.7 Hz, 1H), 6.81 (d, *J* = 8.7 Hz, 1H), 5.72 (s, 2H), 3.81 (s, 3H).

### Example A.3: Synthesis of 3-bromo-1-(2-hydroxy-4-nitrobenzyl)-1H-pyrazole-5-carboxylic acid (3)

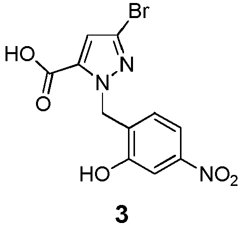

To a solution of NaOH (20 g) in water (800 mL) and methanol (200 mL) compound **2** (50 g; 140 mmol) was added and the mixture was heated at 80 °C for 2 h. The mixture was concentrated to a volume of 300-400 mL, cooled, and acidified to pH 4 with HCl. The precipitate was filtered and dried at 60 °C to yield the desired product **3** (43 g; 90%) as a yellow solid.

¹H NMR (400 MHz, DMSO-*d*₆) δ 10.89 (s, 1H), 7.67 - 7.60 (m, 2H), 7.05 (s, 1H), 6.76 (d, *J* = 8.9 Hz, 1H), 5.73 (s, 2H), COOH not seen.

### Example A.4: Synthesis of 2-[(3-bromo-5-(hydroxymethyl)-1H-pyrazol-1-yl)methyl]-5-nitrophenol (4)

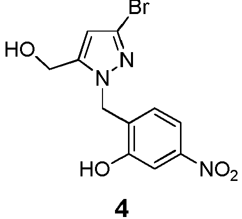

To a solution of **3** (43 g; 126 mmol) in dry THF (900 mL) under argon (CH₃)₂S:BH₃ (67 g; 880 mmol) was added dropwise at 0 °C. The mixture was then stirred at rt for 4 h, warmed to 50°C, stirred for another 10 h, cooled to 0 °C and MeOH (300 mL) was added dropwise. The solvent was evaporated. The residue was diluted with a cold HCl, stirred for 30 min, and extracted with EtOAc. The combined organic phases were dried over Na₂SO₄, filtered and evaporated *in vacuo.* The residue was diluted with hexane, and the precipitate was filtered to yield the desired product **4** (38 g; 92%) as a yellow solid.

¹H NMR (400 MHz, DMSO-*d*₆) δ 10.90 (s, 1H), 7.67 (d, *J =* 2.3 Hz, 1H), 7.64 (s, 1H), 6.91 (d, *J* = 8.5 Hz, 1H), 6.37 (s, 1H), 5.45 (br. s, 1H), 5.32 (s, 2H), 4.51 (s, 2H).

### Example A.5: Synthesis of 5-amino-2-[(3-bromo-5-(hydroxymethyl)-1H-pyrazol-1-yl)methyl]phenol (5)

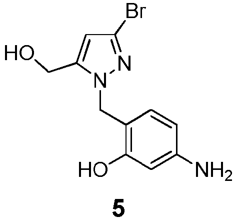

A mixture of **4** (38 g; 116 mmol), NH₄CL (2.5 g), HCL (5 mL), and water (50 mL) was heated to reflux and Fe powder (50 g) was slowly added portion-wise. The heating under reflux was continued for additional 3 h and the hot mixture was filtered. The filtrate was evaporated, diluted with water, and the formed precipitate was filtered off and dried on air to yield the desired product **5** (25 g; 72%) as a colourless glue.

¹H NMR (400 MHz, DMSO-*d*₆) δ 9.24 (s, 1H), 6.62 (d, *J* = 8.1 Hz, 1H), 6.23 (s, 1H), 6.06 (d, *J* = 2.0 Hz, 1H), 5.98 (dd, *J* = 8.3, 2.1 Hz, 1H), 5.37 (t, *J* = 5.6 Hz, 1H), 5.21 - 4.82 (br. s, 4H), 4.52 (d, *J* = 5.4 Hz, 2H).

### Example A.6: Synthesis of tert-butyl [4-((3-bromo-5-(hydroxymethyl)-1H-pyrazol-1-yl)methyl)-3-hydroxyphenyl]carbamate (6)

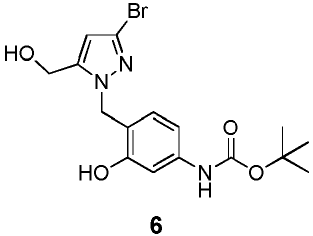

A mixture of **5** (25 g; 84 mmol) in dioxane (400 mL) and Di-t-butyl dicarbonate (20 g; 101 mmol) was heated under reflux overnight. The solvent was distilled off, and the residue was diluted with hexane. The formed precipitate was filtered off and dried to yield the desired product **6** (30 g; 90%).

¹H NMR (400 MHz, DMSO-*d*₆) δ 9.70 (s, 1H), 9.26 (s, 1H), 7.18 (s, 1H), 6.80 - 6.67 (m, 2H), 6.27 (s, 1H), 5.11 (s, 2H), 4.51 (s, 2H), 1.46 (s, 9H), CH₂OH not seen.

### Example A.7: Synthesis of tert-butyl (2-bromo-4,10-dihydropyrazolo[5,1-c][1,4]benzoxazepin-7-yl)carbamate (7)

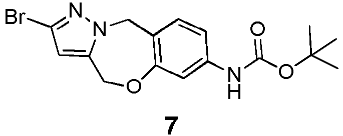

A mixture of **6** (30 g; 75 mmol) in dry THF (300 mL) and PPh₃ (22.4 g; 83 mmol) was cooled to 0 °C and DEAD (15.7 g; 90 mmol) was added dropwise under 15 °C. The mixture was then stirred at rt overnight, upon which the solvents were evaporated in *vacuo,* and the residue purified by column chromatography to yield the desired product **7** (17.4 g; 61%) as an off-white solid.

¹H NMR (400 MHz, DMSO-*d*₆) δ 9.37 (s, 1H), 7.52 (dd, *J* = 5.3, 3.8 Hz, 1H), 7.29 - 7.16 (m, 4H), 7.15 - 7.06 (m, 2H), 6.58 (d, *J* = 0.6 Hz, 1H), 5.56 (s, 2H), 5.34 (s, 2H), 2.44 (s, 3H), 1.46 (s, 9H).

MS (ES) C₁₆H₁₈BrN₃O₃ requires: 379/381, found: 380/382 (M+H)⁺, 100%

### Example A.8: Synthesis of 2-bromo-4,10-dihydropyrazolo[5,1-c][1,4]benzoxazepin-7-amine (8)

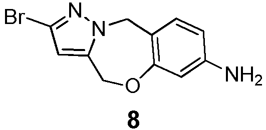

Intermediate **7** (500 mg, 1.315 mmol), was dissolved in a 2:1 mixture of TFA in DCM (15 mL) and stirred overnight. The mixture was diluted with a 2M aq. NaOH solution and extracted with EtOAc. The combined organic phases were dried on MgSO₄, filtered and evaporated *in vacuo* to yield the desired product **8** (370 mg, ~100%) as an off-white solid.

¹H NMR (400 MHz, DMSO-*d*₆) δ 6.96 (d, *J* = 8.1 Hz, 1H), 6.37 (s, 1H), 6.22 (dd, *J =* 8.1, 2.3 Hz, 1H), 6.18 (d, *J* = 2.2 Hz, 1H), 5.31 (s, 2H), 5.21 (s, 2H), 5.18 (s, 2H).

MS (ES) C₁₁H₁₀BrN₃O requires: 279/281, found: 280/282 (M+H)⁺, 99%.

### Example A.9: Synthesis of tert-Butyl [2-(o-tolyl)-4,10-dihydropyrazolo[5,1-c][1,4]benzoxazepin-7-yl]carbamate (12)

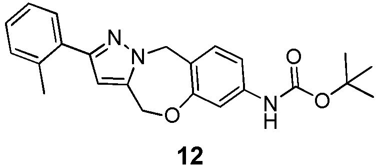

Intermediate **7** (500 mg, 1.32 mmol), o-tolylboronic acid (232 mg, 1.71 mmol), K₂CO₃ (363 mg, 2.63 mmol) and Pd(PPh₃)₄ (304 mg, 0.263 mmol) were suspended in DME/H₂O (2:1, 15 mL) and heated at 120 °C for 1h in a microwave. After cooling to rt, the mixture was diluted with H₂O and extracted with EtOAc. The combined organic phases were dried on MgSO₄, filtered and evaporated *in vacuo.* The crude product was purified by flash chromatography on silica gel using a gradient of EtOAc in cHex to yield the desired product **12** (431 mg, 84%) as a red solid.

¹H NMR (400 MHz, DMSO-*d*₆) δ 9.37 (s, 1H), 7.52 (dd, *J* = 5.3, 3.8 Hz, 1H), 7.29 - 7.16 (m, 4H), 7.15 - 7.06 (m, 2H), 6.58 (d, *J* = 0.6 Hz, 1H), 5.56 (s, 2H), 5.34 (s, 2H), 2.44 (s, 3H), 1.46 (s, 9H).

MS (ES) C₂₃H₂₅N₃O₃ requires: 391, found: 392 (M+H)⁺, 99%.

### Example A.10: Synthesis of 2-(o-tolyl)-4,10-dihydropyrazolo[5,1-c][1,4]benzoxazepin-7-amine (13)

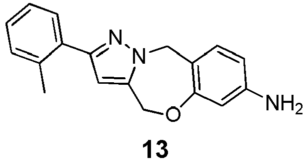

Intermediate **12** (431 mg, 1.10 mmol), was dissolved in a 2:1 mixture of TFA in DCM (10 mL) and stirred for 1 h. The mixture was diluted with sat. NaHCOs solution and extracted with DCM. The combined organic phases were dried on MgSO₄, filtered and evaporated *in vacuo.* The crude product was purified by flash chromatography on silica gel using a gradient of EtOAc in cHex to yield the desired product **13** (320 mg, 100%) as a red solid.

¹H NMR (400 MHz, DMSO-*d*₆) δ 7.55 - 7.47 (m, 1H), 7.26 - 7.15 (m, 3H), 6.99 (d, *J* = 8.2 Hz, 1H), 6.52 (s, 1H), 6.25 -6.13 (m, 2H), 5.41 (s, 2H), 5.26 (s, 2H), 5.16 (s, 2H), 2.44 (s, 3H).

MS (ES) C₁₈H₁₇N₃O requires: 291, found: 292 (M+H)⁺, 99%.

### Example A.11: Synthesis of N-[2-(o-tolyl)-4,10-dihydropyrazolo[5,1-c][1,4]benzoxazepin-7-yl]propanamide (14)

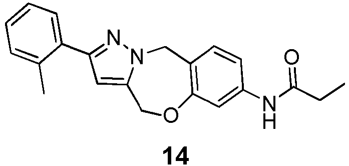

Intermediate **13** (30 mg, 0.124 mmol) was dissolved in dry THF (2 mL), and DIPEA (53 µL, 0.309 mmol), followed by propionyl chloride (10 µL, 0.124 mmol) were added. The reaction was stirred for 1 h 30 min and quenched with MeOH (0.5 mL). The mixture was diluted with water and extracted with EtOAc. The combined organic phases were dried on MgSO₄, filtered and evaporated *in vacuo* to yield the desired product **14** (35 mg, 98%) as an off-white solid.

¹H NMR (400 MHz, DMSO-*d*₆) δ 9.87 (s, 1H), 7.52 (d, *J* = 3.3 Hz, 1H), 7.32 - 7.26 (m, 2H), 7.22 (dd, *J* = 9.7, 6.7 Hz, 3H), 7.17 (d, *J* = 8.3 Hz, 1H), 6.59 (s, 1H), 5.58 (s, 2H), 5.36 (s, 2H), 2.44 (s, 3H), 2.30 (q, *J* = 7.5 Hz, 2H), 1.06 (t, *J* = 7.5 Hz, 3H).

MS (ES) C₂₁H₂₁N₃O₂ requires: 347, found: 348 (M+H)⁺, 99%.

### Example A.12: Synthesis of N-[3-chloro-2-(o-tolyl)-4,10-dihydropyrazolo[5,1-c][1,4]benzoxazepin-7-yl]propanamide (15) and N-[3,8-dichloro-2-(o-tolyl)-4,10-dihydropyrazolo[5,1-c][1,4]benzoxazepin-7-yl]propanamide (16)

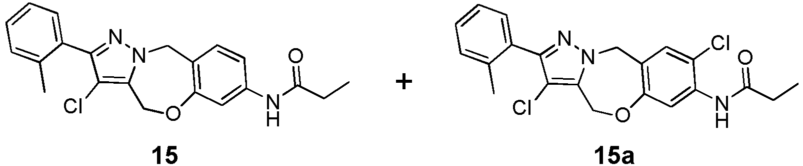

Intermediate **14** (32 mg, 0.092 mmol) was dissolved in DMF (1 mL), and NCS (13 mg, 0.097 mmol) was added. The reaction was heated to 70 °C for 1 h 30 min, upon which the mixture was allowed to cool to rt, diluted with water and extracted with DCM. The combined organic phases were dried on MgSO₄, filtered and evaporated *in vacuo.* The two resulting products were separated and purified by flash chromatography on silica gel using a gradient of EtOAc in cHex to yield the desired product **15** (24 mg, 68%) as an off-white solid, and the dichloro product **15a** (4.1 mg, 11%), as an off-white solid.

**15:** ¹H NMR (400 MHz, DMSO-*d*₆) δ 9.93 (s, 1H), 7.39 (s, 1H), 7.34 - 7.20 (m, 6H), 5.57 (s, 2H), 5.34 (s, 2H), 2.31 (q, *J* = 7.4 Hz, 2H), 2.25 (s, 3H), 1.07 (t, *J* = 7.5 Hz, 3H).

MS (ES) C₂₁H₂₀ClN₃O₂ requires: 381 / 383, found: 382 / 384 (M+H)⁺, 99%.

**15a:** ¹H NMR (400 MHz, DMSO-*d*₆) δ 9.38 (s, 1H), 7.56 (d, *J* = 51.1 Hz, 2H), 7.38 - 7.21 (m, 4H), 5.65 (s, 2H), 5.38 (s, 2H), 2.40 (q, *J* = 7.5 Hz, 2H), 2.24 (s, 3H), 1.08 (t, *J* = 7.5 Hz, 3H).

MS (ES) C₂₁H₁₉Cl₂N₃O₂ requires: 415 / 417, found: 416 / 418 (M+H)⁺, 99%.

Compounds **16-39** constitute further examples according to the present invention that can be obtained following the procedure described for compound **15:** Reference compounds **Ref 1** to **Ref 5** were purchased or prepared following the procedure described for compound **15. Table 1** below lists all compounds and their analytical data (¹H-NMR and LC-MS).

**Table 1. Compounds of the present invention and reference compounds.**

| **#** | **structure** | **¹H-NMR** | **(M+H)⁺** | **LC purity** |
|---|---|---|---|---|
| **Ref 1 (LDC7559)** | 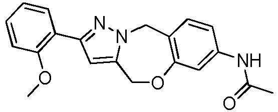 | | 350 | 100% |
| **Ref 2** | 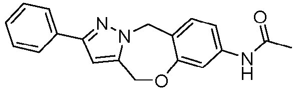 | (400 MHz, Methanol-*d*₄) δ 7.79-7.71 (m, 2H), 7.42-7.33 (m, 3H), 7.27 (dd, 2H), 7.17 (dd, 1H), 6.63 (s, 1H), 5.51 (s, 2H), 5.31 (s, 2H), 2.10 (s, 3H). | 320 | 98% |
| **Ref 3 (NA11)** | 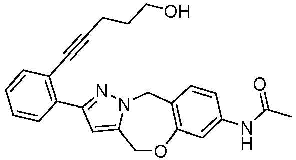 | | 402 | 95% |
| **Ref 4** | 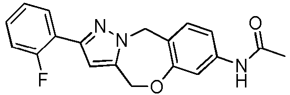 | (400 MHz, DMSO-*d*₆) δ 9.95 (s, 1H), 7.92 (td, 1H), 7.41-7.30 (m, 1H), 7.32-7.20 (m, 4H), 7.15 (dd, 1H), 6.72 (d, 1H), 5.61 (s, 2H), 5.37 (s, 2H), 2.02 (s, 3H). | 338 | 99% |
| **16** | 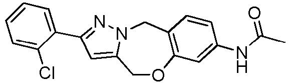 | (400 MHz, DMSO-*d*₆) δ 9.95 (br s, NH), 7.78-7.75 (m, 1H), 7.53-7.50 (m, 1H), 7.41-7.32 (m, 2H), 7.30-7.26 (m, 2H), 7.17-7.13 (m, 1H), 6.82 (s, 1H), 5.61 (s, 2H), 5.38 (s, 2H), 2.02 (s, 3H). | 354 | 96% |
| **17** | 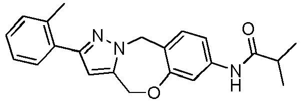 | (400 MHz, Chloroform-*d*) δ 7.48-7.43 (m, 1H), 7.34 (s, 1H), 7.18-7.11 (m, 4H), 7.10-7.03 (m, 2H), 6.23 (s, 1H), 5.44 (s, 2H), 5.20 (s, 2H), 2.43 (sep, 1H), 2.38 (s, 3H), 1.18 (d, 6H). | 362 | 98% |
| **18** | 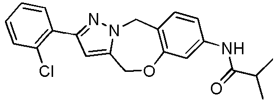 | (400MHz, DMSO-*d*₆) δ 9.84 (br s, NH), 7.78-7.75 (m, 1H), 7.53-7.50 (m, 1H), 7.41-7.27 (m, 4H), 7.32 (m, 1H), 7.22-7.18 (m, 1H), 6.82 (s, 1H), 5.61 (s, 2H), 5.37 (s, 2H), 2.56 (sep, 1H), 1.08 (d, 6H). | 382 / 384 | 91% |
| **19** | 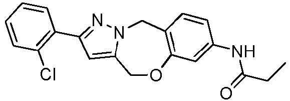 | (400MHz, DMSO-*d*₆) δ 9.88 (br s, NH), 7.78-7-75 (m, 1H), 7.53-7.50 (m, 1H), 7.41-7.26 (m, 4H), 7.19-7.15 (m, 1H), 6.82 (s, 1H), 5.61 (s, 2H), 5.37 (s, 2H), 2.30 (q, 2H), 1.06 (t, 3H). | 368 | 93% |
| **20** | 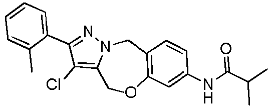 | (400MHz, DMSO-*d*₆) δ 9.90 (br s, NH), 7.42-7.39 (m, 1H), 7.36-7.22 (m, 6H), 5.57 (s, 2H), 5.34 (s, 2H), 2.61-2.53 (m, 1H), 1.09 (d, 6H) | 396/ 398 | 100% |
| **21** | 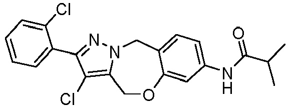 | | 416/ 418 | 100% |
| **22** | 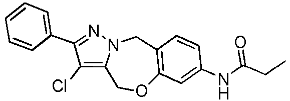 | (400MHz, DMSO-*d*₆) δ 9.95 (br s, NH), 7.84-7.78 (m, 2H), 7.50-7.30 (m, 5H), 7.25-7.20 (m, 1H), 5.59 (s, 2H), 5.34 (s, 2H), 2.31 (q, 2H), 1.07 (t, 3H) | 3681 370 | 100% |
| **23** | 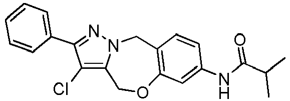 | (400MHz DMSO-*d*₆) δ 9.92 (br s, NH), 7.85-7.78 (m, 2H), 7.52-7.31 (m, 5H), 7.28-7.22 (m, 1H), 5.59 (s, 2H), 5.34 (s, 2H), 2.62-2.54 (m, 1H), 1.09 (d, 6H) | 382 / 384 | 100% |
| **24** | 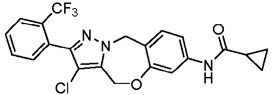 | | 448/ 450 | 100% |
| **25** | 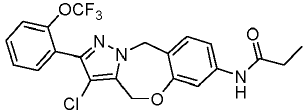 | | 452 / 454 | 100% |
| **26** | 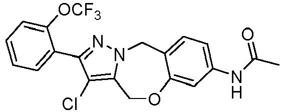 | | 438/ 440 | 100% |
| **27** | 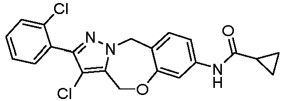 | | 414 / 416 | 96% |
| **28** | 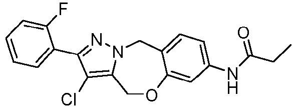 | | 3861 388 | 100% |
| **29** | 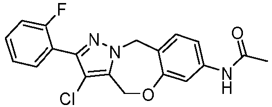 | | 372 / 374 | 99% |
| **30** | 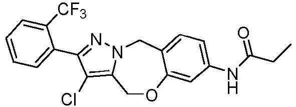 | | 436 / 438 | 98% |
| **31** | 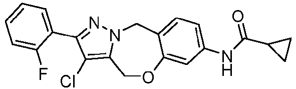 | | 3981 400 | 99% |
| **32** | 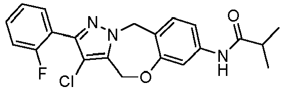 | | 400 / 402 | 99% |
| **33** | 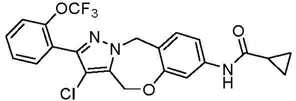 | | 464 / 466 | 99% |
| **34** | 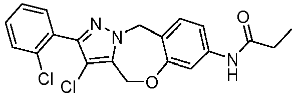 | | 402 / 404 | 98% |
| **35** | 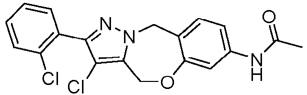 | | 388 / 390 | 88% |
| **36** | 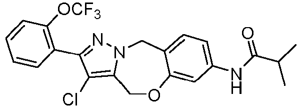 | | 466 / 468 | 98% |
| **37** | 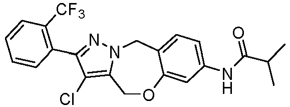 | | 450 / 452 | 98% |
| **38** | 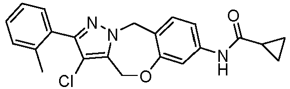 | | 394 / 396 | 97% |
| **39** | 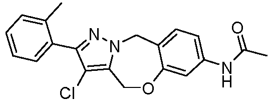 | | 368 / 370 | 96% |
| **40** | 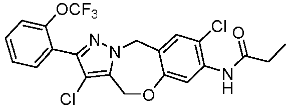 | | 486 / 488 | 99% |
| **41** | 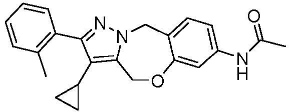 | | 374 | 100% |
| **42** | 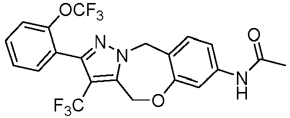 | | 472 | 100% |
| **43** | 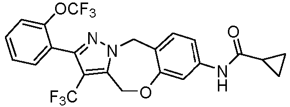 | | 498 | 100% |
| **44** | 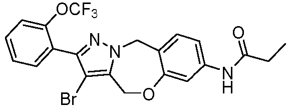 | | 496/49 8 | 100% |
| **45** | 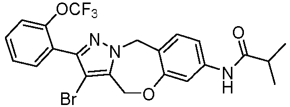 | | 510/51 2 | 100% |
| **46** | 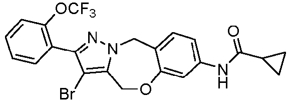 | | 508/51 0 | 99% |
| **47** | 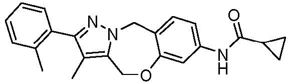 | | 474 | 100% |
| **48** | 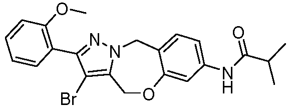 | | 456/45 8 | 100% |
| **49** | 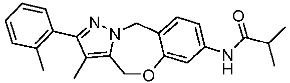 | | 376 | 100% |
| **50** | 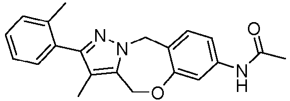 | | 348 | 99% |
| **51** | 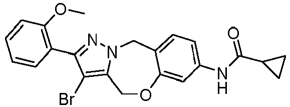 | | 454/45 6 | 100% |
| **52** | 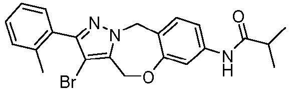 | | 440/44 2 | 100% |
| **53** | 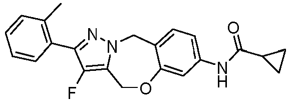 | | 378 | 100% |
| **54** | 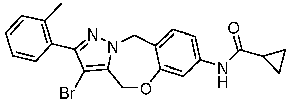 | | 438/44 0 | 100% |
| **55** | 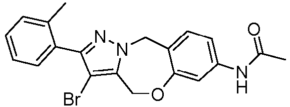 | | 412/41 4 | 98% |
| **56** | 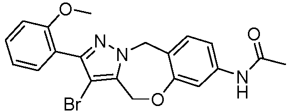 | | 428/43 0 | 100% |
| **57** | 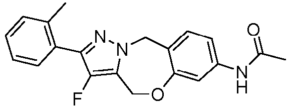 | | 352 | 94% |
| **58** | 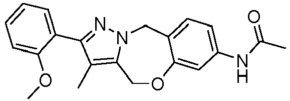 | | 364 | 100% |
| **59** | 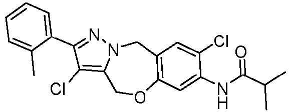 | (400 MHz, DMSO-*d*₆) δ 9.35 (s, 1H), 7.61 (s, 1H), 7.40 (s, 1H), 7.35 - 7.20 (m, 4H), 5.64 (s, 2H), 5.36 (s, 2H), 2.73 (2.76-2.68, m, 1H), 2.22 (s, 3H), 1.08 (d, 6H). | 430/43 2 | 98% |
| **60** | 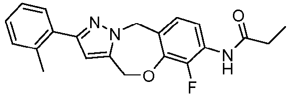 | | 366 | 100% |
| **61** | 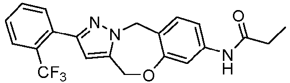 | | 402 | 97% |
| **62** | 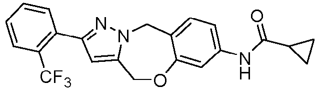 | | 414 | 100% |
| **63** | 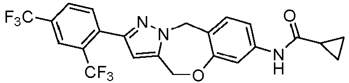 | | 482 | 100% |
| **64** | 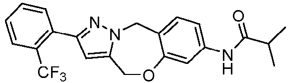 | | 416 | 91% |
| **65** | 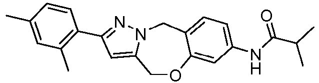 | | 376 | 97% |
| **66** | 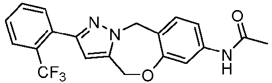 | | 388 | 98% |
| **67** | 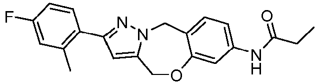 | | 366 | 99% |
| **68** | 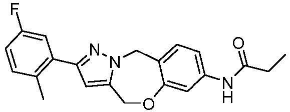 | | 378 | 100% |
| **69** | 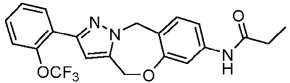 | | 418 | 97% |
| **70** | 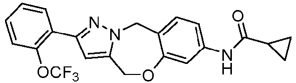 | | 430 | 99% |
| **71** | 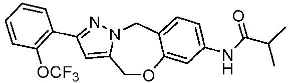 | | 432 | 97% |
| **72** | 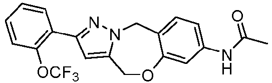 | | | |
| **73** | 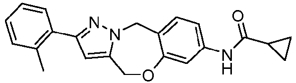 | | 99% | 360 |

### Example B: Fluorometric NET formation assay

### Cell lines & primary blood samples:

If not otherwise stated all materials have been purchased from Sigma Aldrich (Germany). All cell culture media or ingredients were, if not otherwise stated, purchased from Pan Biotech (Aidenbach, Germany). HEK293T and THP1 cell lines has been purchased from DSMZ and and ATCC, respectively. Both were cultivated according to the supplier's recommendations. hPBMCs were provided by Blutspendedienst West (Institut Hagen; Deutsches Rotes Kreuz). Neutrophils were purified from human healthy donors after assessment of any either chronic or active infection and after signing an informed consent according to German law terms.

### Neutrophil preparation:

Human blood samples were drawn and subjected to neutrophil preparation as follows. 1h after blood drawing a Histopaque1119 (Sigma) gradient was performed (i.e. 20 mL blood together with 20 mL Histopaque). To separate neutrophils from contaminating hPBMCs and erythrocytes neutrophil-rich portion was transferred to a new tube and washed with PBS containing 0.5% human serum albumin. Finally, a Percoll gradient (65% - 85% Percoll; VWR) was used for further elimination of erythrocytes. Cells were again washed with PBS containing 0.5% human serum albumin, counted and seeded in black, clear bottom 384well-plates (Greiner µclear; 15000 cells/well; assay medium: RPMI1640 + 10 mM Hepes, phenolred free, FCS free).

### Assay description:

Purified neutrophils were allowed to accommodate for 30 min at 37 °C, 5% CO2 in above mentioned plates. Compounds were added as DMSO solutions either by manual pipetting or by using an Echo dispensing device (Labcyte Inc.). Subsequently, NET formation was started by addition of PMA (final conc. 40 nM). After an incubation of 4h in a humidified chamber at 37 °C and 5% CO2 samples were then stained with Sytox-Green (Thermofisher Scientific; final concentration 2.5 µM) and subjected to fluorescence measurements using a PerkinElmer Victor X5. Unstimulated neutrophils were used as negative control (0% NET formation). PMA-treated ones represented positive control (100% NET formation). Compound action was derived as percentage of positive control values. Data was finally analyzed using XLFIT Plugin (dose response Fit 205) for IC50 determinations.

### Example C: LDH Glo Assay upon GSDMD NT transfection

On day 1 HEK293T cells were detached and adjusted to a concentration of 8x105 cells/ml in growth medium. Cells were transfected in suspension by addition of 50 µL of premixed transfection solution (1µg plasmid coding for the N-terminal part of GSDMD [accession numbers: NM_024736.1 to .3; amino acids 1-275], 3 µL TransIT^{®}-LT1 [MoBiTec] filled up with Opti-MEM Reduced Serum Medium) per ml of cell suspension. Cells were directly seeded in 384well plates (Greiner, white) at a density of 20.000 cells/well in 25 µL medium. Transfected cells were allowed to incubate for 2h and then treated with compounds by an Echo dispensing device. After 22h of further incubation, LDH release war determined as described above. Mock transfected wells served a negative control (0% LDH release/GSDMD activity), those transfected with GSDMD N-terminus coding plasmids represented positive control samples (100% LDH release/GSDMD activity). Compound action was derived as percentage of positive control values. Data was finally analyzed using Scigilian Analyze platform employing a four-parameter fit (Y=Min+(Max-Min)/(1+((10 Log1 P)/X)^Slope )) for IC₅₀ determinations.

### Example D: ROS-Glo^{™} H₂O₂ Assay

The ROS-Glo^{™} H₂O₂ assay is based on the detection of H₂O₂, a reactive oxygen species (ROS), through a luciferin derivative that reacts with H₂O₂to generate a luciferin precursor and thereby, luminescence corresponding to the H₂O₂levels in the cells.

The isolation of neutrophils from buffy coats using a method based on density gradient centrifugation. Briefly, Histopaque-1119 is added to the peripheral blood and centrifuged at 800 x g to separate the neutrophils from red blood cells (RBCs), plasma and other cellular contents. The neutrophils are then collected and washed with DPBS + 20% Albumin, followed by centrifugation at 300 x g. A Percoll^{™} Plus gradient (65-85%) is then used to collect the neutrophils in the interphase between the 65-70% and 70-75% gradients following centrifugation. The isolated neutrophils are then used further in the ROS-Glo^{™} H₂O₂Assay.

For the ROS-Glo^{™} H₂O₂assay, neutrophil culture medium (RPMI 1640 + 10mM HEPES) is added to 384 well plates. Compounds and controls are dosed onto the medium plate. Menadione and DMSO are dosed on the plates as C- and C+, respectively. Following compound dosing, 15.000 neutrophils/well are added to all wells and incubated with the compounds for 90 minutes. 30 minutes prior to the end of the 90 minutes compound treatment, the H₂O₂Substrate provided with the kit is added to all wells and incubated for 30 minutes with the neutrophils + compounds. Following this, Phorbol 12-myristoyl-13-acetyl (PMA) to a final concentration of 50 nM is added per well and the plates incubated a further 60 minutes. At the end of the 60 minutes, the ROS-Glo^{™} H₂O₂Detection Solution prepared as per the manufacturers instructions is added to the plates, incubated for 20 mins and the luminescence read-out measured with the Viktor.

### Example E: Biological activities of compounds

Activities of compounds are listed in **Table 3** as determined by the Sytox-Green staining assay for NET formation inhibitory activity according to Example 4 and the of blocking the Gasdermin-D pore function (GSDMD assay according to Example 5) were grouped according in a way that is described in **Table 2.** Additionally, the results from a ROS detection assay, performed as described in example E, are reported. The data for inventive ortho-chloro-phenyl analogues and CI-pyrazoles analogues are compared to the published analogues **Ref 1** (LDC7559) and **Ref 5,** the compounds of WO 2023/287793 A1 **Ref 2** (ex.1), **Ref 3** (ex.6, NA-11), and **Ref 4** (ex.55), as well as NADPH oxidase inhibitors menadione and DPI (Diphenyleneiodonium chloride).

**Table 2: Activity ranges**

| | | | | |
|---|---|---|---|---|
| NET Formation assay Sytox Green staining (average IC₅₀) | < 1 µM | 1 µM ≤ x < 3 µM | 3 µM ≤ x< 10µM | > 10 µM |
| | +++ | ++ | + | |
| GSDMD (IC₅₀) | < 100 nM | 100nM ≤ x < 1µM | 1 µM ≤ x < 4µM | > 10 µM |
| | +++ | ++ | + | - |
| NET_ROS-H₂O₂-Glo_Neutrophil (IC₅₀) | < 10 µM | 10 µM ≤ x < 20 µM | 20 µM ≤ x < 30 µM | > 30 µM |
| | - | + | ++ | +++ |

**Table 3: Biological activities (NET formation, GSDMD assay, and ROS detection)**

| **#** | **NET Formation assay Sytox Green staining (average IC₅₀) (µM)** | **GSDMD N-term_LDH-release_Hek293T_24h (average IC₅₀) (µM)** | **NET_ROS-H₂O₂**-**Glo Neutrophil 384_ 1hr_IC₅₀ (IC₅₀) (µM)** |
|---|---|---|---|
| **Ref 1** | 2.31 (++) | 3.49 (+) | 22.5 (++) |
| **Ref 2** | 2.16 (++) Lit: 0.19 | 10.37 (-) | 20.7 (++) |
| **Ref 3** | 0.49 (+++) Lit: 0.004 | 23.43 (-) | >30 (+++) |
| **Ref 4** | 4.22 (+) | 3.23 (+) | >30 (+++) |
| **14** | ++ | ++ | |
| **15** | +++ | +++ | +++ |
| **16** | 1.33 (++) | 0.415 (++) | 23.9 (++) |
| **17** | 0.855 (+++) | 0.32 (++) | >30 (+++) |
| **18** | 1.15 (++) | 0.319 (++) | >30 (+++) |
| **19** | 3.17 (+) | 0.18 (++) | 16.8 (+) |
| **20** | ++ | ++ | ++ |
| **21** | ++ | +++ | ++ |
| **22** | +++ | ++ | +++ |
| **23** | +++ | ++ | +++ |
| **24** | ++ | ++ | +++ |
| **25** | +++ | +++ | ++ |
| **26** | +++ | +++ | + |
| **27** | +++ | ++ | + |
| **28** | +++ | ++ | +++ |
| **29** | ++ | ++ | + |
| **30** | +++ | +++ | +++ |
| **31** | +++ | ++ | +++ |
| **32** | +++ | ++ | +++ |
| **33** | ++ | +++ | +++ |
| **34** | ++ | +++ | ++ |
| **35** | ++ | ++ | + |
| **36** | ++ | +++ | +++ |
| **37** | ++ | ++ | ++ |
| **38** | ++ | ++ | +++ |
| **39** | +++ | ++ | ++ |
| **40** | + | ++ | +++ |
| **41** | ++ | +++ | |
| **42** | ++ | +++ | |
| **43** | + | +++ | |
| **44** | + | ++ | |
| **45** | - | ++ | |
| **46** | - | ++ | |
| **47** | ++ | ++ | |
| **48** | +++ | ++ | |
| **49** | ++ | ++ | |
| **50** | ++ | ++ | |
| **51** | +++ | ++ | |
| **52** | +++ | ++ | +++ |
| **53** | ++ | ++ | |
| **54** | +++ | ++ | +++ |
| **55** | +++ | ++ | + |
| **56** | +++ | ++ | |
| **57** | ++ | ++ | ++ |
| **58** | + | ++ | |
| **59** | + | ++ | +++ |
| **60** | +++ | ++ | |
| **61** | +++ | ++ | |
| **62** | | ++ | |
| **63** | + | ++ | |
| **64** | ++ | ++ | |
| **65** | +++ | ++ | |
| **66** | + | ++ | |
| **67** | +++ | ++ | |
| **68** | ++ | ++ | |
| **69** | +++ | ++ | |
| **70** | ++ | ++ | |
| **71** | - | ++ | |
| **72** | - | ++ | |
| **73** | ++ | ++ | |
| menadione | | | 1.53 |
| DPI | 0.0558 | | 0.0492 |

### Conclusion

Genetic and pharmacological evidence supports Gasdermin D (GSDMD) as a critical target for neutrophil extracellular trap (NET) formation. Knockout studies of GSDMD have demonstrated significant effects on NET formation both in vitro and in vivo, as reported in Chen et al., 2018 (DOI: 10.1126/sciimmunol.aar6676). Furthermore, pharmacological inhibition of GSDMD using disulfiram has been shown to modulate NET formation in experimental models, both in vitro and in vivo, as highlighted in recent studies (e.g., DOI: 10.1172/jci.insight.157342).

To date, no evidence exists that modulation of other potential targets, such as PFKL (via knockout or overexpression), directly results in the inhibition of NET formation.

Experimental assay data above reveal a correlation between the activity of the inventive compounds in NET formation assays and their inhibition of GSDMD N-terminal (GSDMD-NT) activity. In contrast, no correlation is observed between NET formation and reactive oxygen species (ROS) assays. This distinction underscores the selectivity of these compounds for GSDMD-mediated pathways over ROS-dependent mechanisms.

In contrast, reference compounds may exhibit activity on targets other than GSDMD, such as PFKL. For example, Reference compound 3 demonstrates minimal activity in GSDMD-NT assays and is thus considered an outlier in the context of NET formation and GSDMD-NT inhibition.

The mechanistic role of GSDMD in NET formation positions it as a critical target for therapeutic intervention in diseases driven by excessive or dysregulated NET release. Its specificity, combined with the growing body of genetic and pharmacological evidence, highlights its therapeutic promise in mitigating inflammatory and autoimmune disorders, cardiovascular diseases, and sepsis. By precisely regulating NET formation, GSDMD inhibition offers a novel and targeted strategy to reduce pathological inflammation while minimizing off-target effects.

### Example F: Acute Ischemia Reperfusion Rat Model of Middle Cerebral Artery Occlusion

### Purpose

MCAO is the sudden onset of a focal neurologic deficit resulting from brain infarction in the territory supplied by the middle cerebral artery (MCA). In humans, MCAO is the most common cause of cerebral ischemia stroke. The study was to evaluate the effects of the test compounds on ischemic stroke in a rat model of transient MCAO.

### Study Design

80 male Sprague-Dawley rats (200g±20g) were used for this study. Rats were acclimatized in the animal facility for one week before experiment. Animals were kept under a standard condition with room temperature at 21-23°C, 30-70% relative humidity, and a 12:12h light: dark cycle. Chow and water were available ad libitum. All the *in vivo* experimental procedures were approved by the institutional animal care and use committee (IACUC) at HDB.

### Introduction

The study was to evaluate effects of the test compounds on ischemic stroke in a rat model of transient MCAO. The study was conducted by HDB at its facility. The study is conducted at AAALAC accredited facility and all animal study procedures are approved by the Institutional Animal Care and Use Committee (IACUC) of HDB.

### Materials and Methods

This study is conducted according to the procedures outlined below and as described in the protocol section.

**Table 4: Consumables and Equipment**

| **Equipment** | **Manufacturer** | **Catalogue No.** |
|---|---|---|
| IRIS-Fine scissors | RWD | S12009-11 |
| Suture embolization | RWD | MSRC37B200PK |
| VANNAS | RWD | S11003-08 |
| Surgical sutures | Shanghai Jinhuan Medical | KC439 |
| Centrifuge | Eppendorf | Model 5810R |
| Balance | Shanghai JINGHAI | Model YP1002N |
| EDTA anticoagulation tube | BD | 367861 |

**Table 5: Reagents**

| **Name** | **Manufacture** | **Cat#** |
|---|---|---|
| Edaravone | MCE | HY-B0099 |

### Formulation Preparation

### Vehicle Preparation

A total volume of 30 mL was achieved by combining 1.5 mL of DMSO, 3 mL of Solutol HS15, and 25.5 mL of PBS.

### Preparation of 16

Accurately weighted out 30.00 mg of **16,** followed by the addition of 1.5 mL of DMSO, ensuring thorough mixing. Subsequently, incorporated 3 mL of Solutol HS15, mixing adequately. Ultimately, supplement the solution with PBS to achieve a total volume of 30 mL, ensuring homogeneity through vigorous vortexing and ultrasonic waves. The resultant concentration is 1 mg/mL, with a dosage of 10 mL/kg. The formula was freshly prepared daily.

### Edaravone Preparation

Dexamethasone: dosing level, 3 mg/kg; concentration, 0.3 mg/mL; dosing volume, 10 mL/kg.

Accurately weighted out 9.00 mg of Edaravone, and added saline to 30 mL, mixed well by vigorous vortex and sonication, and the stock solution was 3 mg/mL (freshly prepared daily).

### Animals

### Animal Order

80 male Sprague-Dawley rats (200g±20g) were purchased. Animals were acclimatized for 1 week prior to the experiment. All the *in vivo* experimental procedures were approved by the institutional animal care and use committee (IACUC) at HDB. All euthanasia was performed using anesthesia and all efforts were made to minimize animal suffering.

### Animal Reception, Health Examination and Acclimation

The experimental animals were placed in the breeding cages immediately after being received. Afterwards, each mouse will undergo a health examination, including appearance, limbs, nose and mouth, etc. In addition, the abnormal shape or abnormal activity of each animal was checked.

### Animal Breeding

This protocol (AUF# 202) is approved by the HDB Animal Care and Use Committee before study initiation. Animals were group-housed (5 animals per cage) with bedding under controlled temperature (21-23°C), noise, humidity (30-70%), and lighting (12 h light and 12 h dark) conditions. All animals have free access to purified water and standard certified rodent chow. The animal facility of HD Biosciences has been accredited by AAALAC. The use of experimental animals has been approved by the Shanghai Science and Technology Committee (STCSM), and the license number is SCXK (Shanghai) 2017-0005 and SCXK (Beijing) 2019-0010.

### Animal Feed and Water

All animals can eat freely during the implementation of the project, and the feed was purchased from Beijing Keao Xieli Feed Co., Ltd. The feed supplier will provide a quality certificate for each batch of feed. HDB entrusts a third party to test the feed once a year and has a corresponding feed test report. All certificates are archived by HDB.

All animals can drink freely during the execution of the project. All drinking water is tap water filtered and sterilized by Mol ultrapure water machines. The water quality test is carried out once a year, and the test items include heavy metals, nitrates, water-soluble minerals, the total number of colonies, and the number of Escherichia coli. The inspection certificate is archived by HDB.

There is no evidence that known contaminants in food and drinking water interfere with this study.

### Breeding Cages and Animal Numbers

Each animal is assigned a unique number. Before animals are grouped, the breeding cages are equipped with information cards. The information on the cards includes item number, animal species, gender, cage number and animal number.

After the animals are grouped, replace the cage with a new information card. Different groups use information cards of different colors to distinguish them. The information contained on the information cards is the same as before.

The grouping information was recorded in detail in the random grouping record section.

The rearing cages are placed on the rearing racks in layers to reduce the impact of the environment on this study.

### Experimental Design

### Group Design

Group information is listed in **Table 6** . After a 7-day adaptation period, 80 male Sprague-Dawley rats were randomly divided into 8 groups, with 10 animals in each group, according to their body weight. Only the groups relevant to this invention are depicted in the table below. Compounds were applied 30 minutes before the removal of the occlusion.

**Table 6: Group information**

| **Number** | **Group** | **Animal Number** | **Rat Strain** | **Gender** | **Compound Treatment** | **Dosing Level** | **Dosing Time** |
|---|---|---|---|---|---|---|---|
| 1 | Sham-surgery | 10 | SD | M | Vehicle | N/A; IP | -0.5 h |
| 2 | MCAO | 10 | SD | M | Vehicle | N/A; IP | -0.5 h |
| 3 | MCAO | 10 | SD | M | Edaravone | 3 mpk; IP | -0.5 h |
| 5 | MCAO | 10 | SD | M | **16** | 10mg/kg; IP 5% DMSO, 10% Solutol HS15, 85% PBS | -0.5 h |

### MCAO model development (n=10 per group):

1. Under anesthesia, the right common carotid artery (CCA), internal carotid artery (ICA), and external carotid artery (ECA) were exposed through a midline incision of the neck.
2. A commercial monofilament (silicon-coated) was used as an occluder and inserted via the ECA.
3. The occluder was advanced into the ICA 18 ± 0.5 mm beyond the carotid bifurcation.
4. Mild resistance indicates that the occluder is properly lodged in the anterior cerebral artery and blocks blood flow to the middle cerebral artery (MCA).
5. After 120 minutes, reperfusion was allowed by withdrawing the monofilament by approximately 10 mm.
6. Body temperature was kept around 36.5 °C with a heading pad during the surgery process.

### Clinical signs were monitored and evaluated including (n=10):

0 - no neurological deficit;
1 - failure to extend left forepaw fully;
2 - inconstant circling to the left;
3 - constant circling to the left;
4 - falling to the left;
5 - no spontaneous walking with depressed level of consciousness;
6 - death.

### Experimental Schedule

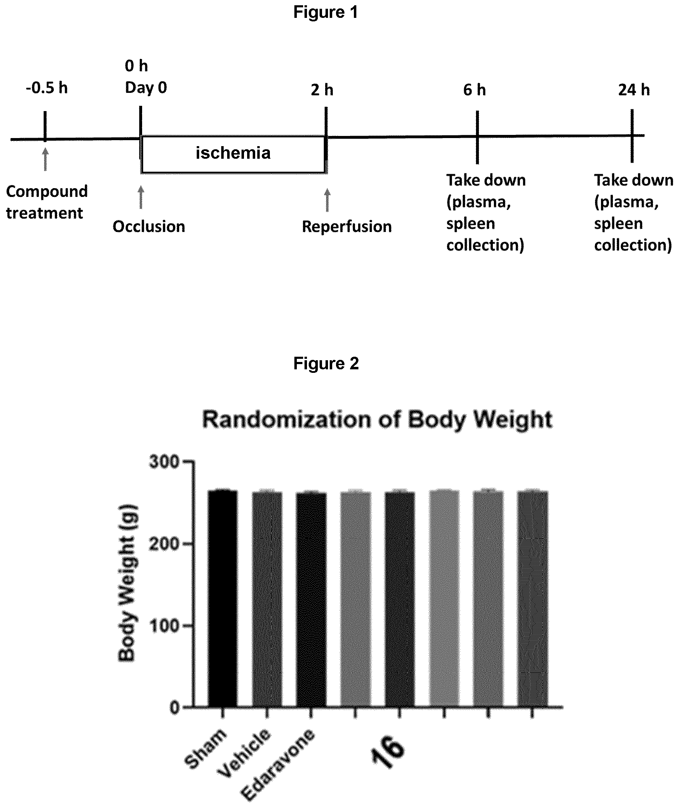

The whole project was strictly conducted according to the timetable listed in **Figure 1****.**

### Compound Treatment

A positive control, namely an injectable solution of 3 mg/kg 3-Methyl-1-phenyl-2-pyrazoline-5-one (MCI-186 / Edaravone), was administered 30 minutes prior to occlusion. A control group was established by administering an intraperitoneal injection of 0.9% saline solution. **16** was administered 30 minutes prior to the occlusion.

### Blood Collection

At the study endpoints (6 h and 24 h post-surgery), rats were euthanized by carbon dioxide (CO₂). 50% of the animals were sacrificed after 6 hours, and the remaining 50% were sacrificed after 24 hours. Plasma samples were collected through a cardiac puncture and put into EDTA-coated tubes (centrifuged at 8000 g for 10 min). The plasma samples were temporarily stored at -80°C and then shipped to the inventors upon termination.

### Tissue Collection

At the study endpoints (6 h and 24 h post-surgery), spleen samples were collected. After blood collection, all animals were perfused with normal saline, and the spleen samples were collected. Then spleen tissues were fixed in 10% NBF for at least 24 hours and embedded in paraffin after dehydration. Also, spleen & brain FFPE were shipped to the inventors.

### Cerebral infract size measurement

After neurological evaluation, half of the rats in each group were sacrificed at 6 hours of reperfusion, and the brains were removed quickly. Half of the rats in each group were sacrificed at 24 hours of reperfusion, and the brains were removed and frozen quickly. 2-mm coronal sections were stained with 2% 2,3,5-triphenyl tetrazolium chloride (TTC) at 37°C for 15 minutes, and then fixed with 10% NBF for 30 minutes. Infarcted area outlined in white and the whole brain section area were measured by ZEISS Zen 3.4 software. The infarcted size rate was calculated.

### Statistical Analysis

Results were expressed as mean ± SEM. Statistical analysis was performed using a one-way ANOVA, if significant, followed by a post-hoc Dunnett's test. Non-parametric tests like Mann-Whitney were used when the data did not follow a Gaussian distribution. The difference was considered significant at p < 0.05.

### Results

### Randomization Before Compound Treatment

All animals were acclimatized for one week before the experiment and randomized on day 0 according to body weight (**Figure 2**). Then the compounds treatments were started.

### Neurological Deficits Score

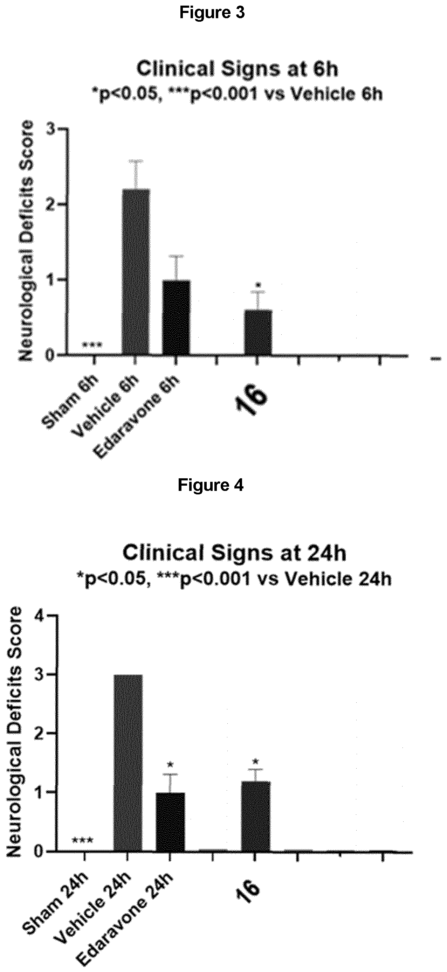

Neurological deficit scores were used to assess the extent of neurological damage in each group. The higher the neurological deficit score, the more severe the impairment of motor motion. As shown in **Figure 3**, animals in the MCAO group showed significantly more neurological deficits than the sham group at 6 hours after ischemia (*p*<0.001), indicating a severe neurologic injury. However, the MCAO rats pretreated with **16** had significantly less neurological impairment (*p*<0.05) (**Figure 3**).

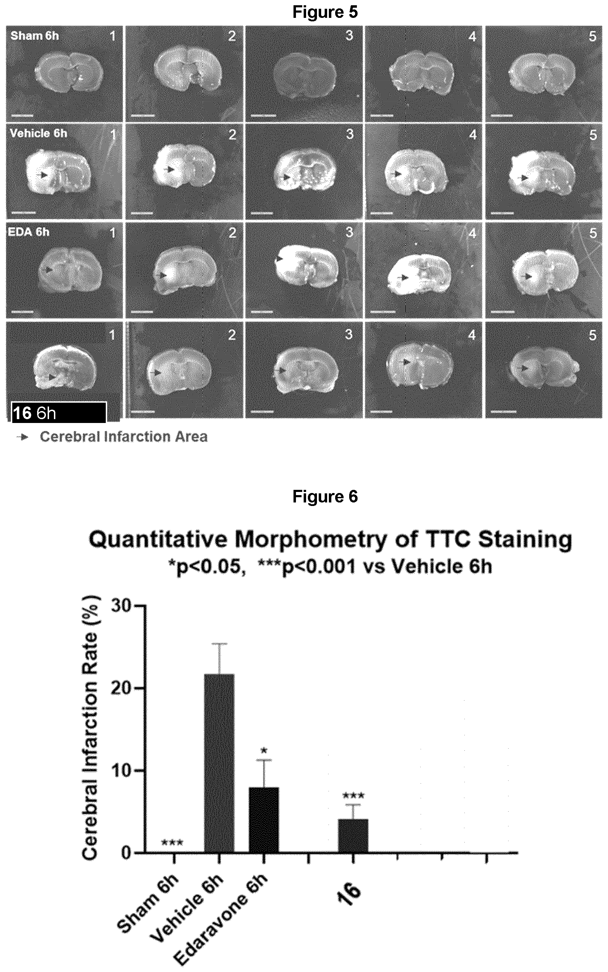

As shown in **Figure 5****,** animals in the MCAO group showed significantly more neurological deficits than the sham group at 24 hours after ischemia (p<0.001), indicating a severe neurologic injury. However, the MCAO rats pretreated with Edaravone had significantly less neurological impairment at 24 hours after ischemia (*p*<0.05) (Figure **5**). The MCAO rats pretreated with **16** had significantly less neurological impairment at 24 hours after ischemia (p<0.05) (**Figure 4**).

### infarction Size at 6 Hours Post-Ischemia

Infarct size at 6 h post-ischemia was measured in order to evaluate the test compounds' neuroprotective effects. About 2% TTC was used to calculate the infarct size. Infarct size was presented as a percentage of the infarct to the whole brain section area. Red areas represent normal tissue, while white areas represent infarction. As shown in **Figure 5** and **Figure 6****,** TTC staining showed that the infarct size was larger in the MCAO group than that in the sham group, whereas administration with Edaravone obviously reduced the infarct volume of rats challenged with MCAO by 14% (*p*<0.05). **Figure 5** and **Figure 6** showed that the infarct size in the **16** group was significantly lower than the MCAO group by 18% (*p*<0.001) **(****Figure 5** and **Figure 6****).**

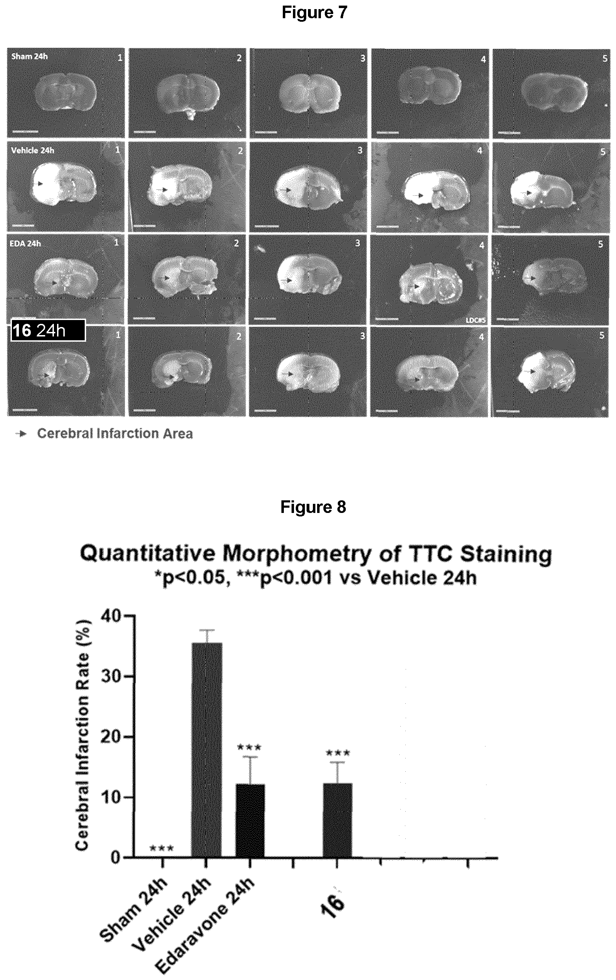

A 2-h MCAO followed by 22-h reperfusion induced an infarct size of 35 ± 2% in vehicle-treated rats (**Figure 7** and **Figure 8**). In contrast, treatment with Edaravone before ischemia reduced infarct size significantly by approximately 23% (*p*<0.001) (**Figure 7** and **Figure 8**). The MCAO rats pretreated with **16** had a significantly decreased infarct area compared to rats pretreated with vehicle (*p*<0.001) (**Figure 7** and **Figure 8**)**.**

In this study, a MCAO rat model was successfully established. Compared to the normal control group, MCAO rats significantly increased their neurological deficit score and infarct area.

Compared to the vehicle group, the MCAO rats pretreated with Edaravone (3 mpk, i.p.) had a decreased tendency on the neurological deficit score at 6 hours post-ischemia, but there were no significant differences. However, there was a significant reduction in the neurological deficit score at 24 hours post-ischemia. Compared to the model group, treatment with Edaravone (3 mpk, i.p.) significantly reduced the infarct area at 6 and 24 hours post-ischemia.

Compared to the vehicle group, the MCAO rats pretreated with **16** (10 mpk, i.p.) significantly decreased their neurological deficit score and infarct area at 6 and 24 hours post-ischemia.

In conclusion, **16** showed efficacy in MCAO rat models at 6 and 24 hours post-ischemia.

## Claims

1. A compound of general formula (**I**) 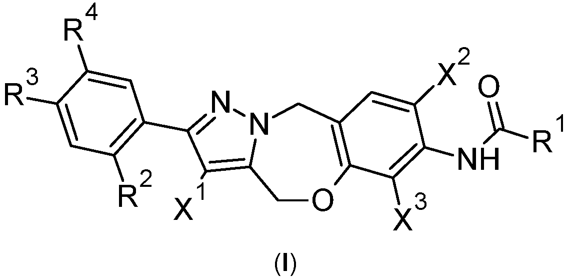 wherein
X¹ represents -H -CH₃, -C₂H₅, -C₃H₇, -CH(CH₃)₂, -cyclo-C₃H₅, -F, -Cl, -Br, or -CF₃;
X² and X³ are independently of each other selected from -H, -F, -Cl, and -Br ;
R¹ represents -CH₃, -C₂H₅, -C₃H₇, -CH(CH₃)₂, or -cyclo-C₃H₅;
R² represents -H, -F, -Cl, -CH₃, -C₂H₅, -C₃H₇, -CH(CH₃)₂, -CF₃, -OCF₃, -CN, -OH, or -OCH₃;
R³ and R⁴ are independently of each other selected from -H, -F, -CH₃, and -CF₃ ;
with the proviso that when X¹, X², and X³ are simultaneously -H, R² represents -Cl, -CF₃, or -OCF₃;
or enantiomers, mixtures of enantiomers, diastereoisomers, mixtures of diastereoisomers, hydrates, solvates, racemates or pharmaceutically acceptable salts thereof.

2. The compound according to claim 1, wherein R³ and R⁴ represent -H.

3. The compound according to claim 1 of general formula (**II**), 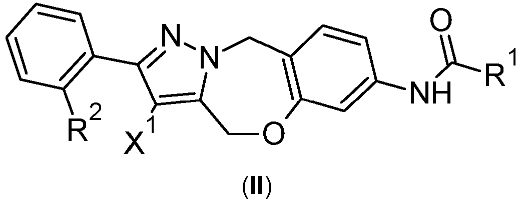 wherein X¹, R¹ and R² have the meanings as defined in claim 1, wherein when X¹ represents -H, R² represents -Cl, -CF₃, or -OCF₃.

4. The compound according to claim 1 of general formula (**II-D**), 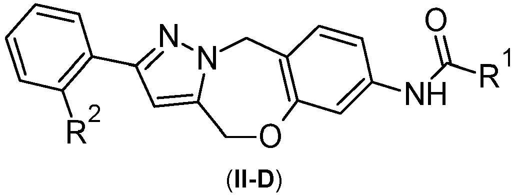 wherein
R² represents -Cl, -CF₃, or -OCF₃; and
R¹ has the meanings as defined in claim 1.

5. The compound according to claim 1 of the general formula (**II-E**) 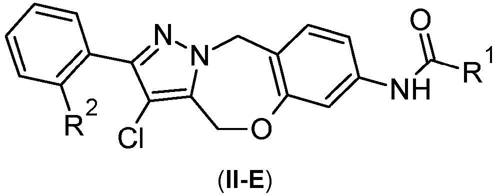 wherein
R² represents -H, -F, -Cl, -CH₃, -C₂H₅, -C₃H₇, -CH(CH₃)₂, -CF₃, -OCF₃, -CN, -OH, or -OCH₃; and R¹ has the meanings as defined in claim 1.

6. The compound according to claim 1 selected from:
N-[2-(2-chlorophenyl)-4,10-dihydropyrazolo[5,1-c][1,4]benzoxazepin-7-yl]acetamide,
N-[2-(2-chlorophenyl)-4,10-dihydropyrazolo[5,1 -c][1,4]benzoxazepin-7-yl]-2-methyl-propanamide,
N-[2-(2-chlorophenyl)-4,10-dihydropyrazolo[5,1-c][1,4]benzoxazepin-7-yl]propanamide,
N-[3-chloro-2-(o-tolyl)-4,10-dihydropyrazolo[5,1-c][1,4]benzoxazepin-7-yl]propanamide,
N-[3-chloro-2-(o-tolyl)-4,10-dihydropyrazolo[5,1 -c][1,4]benzoxazepin-7-yl]-2-methyl-propanamide,
N-(3-chloro-2-(2-chlorophenyl)-4H,10H-benzo[f]pyrazolo[5,1-c][1,4]oxazepin-7-yl)isobutyramide,
N-(3-chloro-2-phenyl-4,10-dihydropyrazolo[5,1-c][1,4]benzoxazepin-7-yl)propanamide,
N-(3-chloro-2-phenyl-4,10-dihydropyrazolo[5,1 -c][1,4]benzoxazepin-7-yl)-2-methyl-propanamide,
N-(3-chloro-2-(2-(trifluoromethyl)phenyl)-4H,10H-benzo[f]pyrazolo[5,1-c][1,4]oxazepin-7-yl)cyclopropanecarboxamide,
N-(3-chloro-2-(2-(trifluoromethoxy)phenyl)-4H,10H-benzo[f]pyrazolo[5,1-c][1,4]oxazepin-7-yl)propionamide
N-(3-chloro-2-(2-(trifluoromethoxy)phenyl)-4H,10H-benzo[f]pyrazolo[5,1-c][1,4]oxazepin-7-yl)acetamide,,
N-(3,8-dichloro-2-(2-(trifluoromethoxy)phenyl)-4H,10H-benzo[f]pyrazolo[5,1-c][1,4]oxazepin-7-yl)propionamide,
N-(3-chloro-2-(2-chlorophenyl)-4H,10H-benzo[f]pyrazolo[5,1-c][1,4]oxazepin-7-yl)cyclopropanecarboxamide,
N-(3-chloro-2-(2-fluorophenyl)-4H,10H-benzo[f]pyrazolo[5,1-c][1,4]oxazepin-7-yl)propionamide,
N-(3-chloro-2-(2-fluorophenyl)-4H,10H-benzo[f]pyrazolo[5,1-c][1,4]oxazepin-7-yl)acetamide,
N-(3-chloro-2-(2-(trifluoromethyl)phenyl)-4H,10H-benzo[f]pyrazolo[5,1-c][1,4]oxazepin-7-yl)propionamide,
N-(3-chloro-2-(2-fluorophenyl)-4H,10H-benzo[f]pyrazolo[5,1-c][1,4]oxazepin-7-yl)cyclopropanecarboxamide,
N-(3-chloro-2-(2-fluorophenyl)-4H,10H-benzo[f]pyrazolo[5,1-c][1,4]oxazepin-7-yl)isobutyramide,
N-(3-chloro-2-(2-(trifluoromethoxy)phenyl)-4H,10H-benzo[f]pyrazolo[5,1-c][1,4]oxazepin-7-yl)cyclopropanecarboxamide,
N-(3-chloro-2-(2-chlorophenyl)-4H,10H-benzo[f]pyrazolo[5,1-c][1,4]oxazepin-7-yl)propionamide,
N-(3-chloro-2-(2-chlorophenyl)-4H,10H-benzo[f]pyrazolo[5,1-c][1,4]oxazepin-7-yl)acetamide,
N-(3-chloro-2-(2-(trifluoromethoxy)phenyl)-4H,10H-benzo[f]pyrazolo[5,1 c][1,4]oxazepin-7-yl)isobutyramide,
N-(3-chloro-2-(2-(trifluoromethyl)phenyl)-4H,10H-benzo[f]pyrazolo[5,1-c][1,4]oxazepin-7-yl)isobutyramide,
N-(3-chloro-2-(o-tolyl)-4H,10H-benzo[f]pyrazolo[5,1-c][1,4]oxazepin-7-yl)cyclopropanecarboxamide,
N-(3-chloro-2-(o-tolyl)-4H,10H-benzo[f]pyrazolo[5,1-c][1,4]oxazepin-7-yl)acetamide,
N-(3,8-dichloro-2-(2-(trifluoromethoxy)phenyl)-4H,10H-benzo[f]pyrazolo[5,1-c][1,4]oxazepin-7-yl)propionamide,
N-[3-cyclopropyl-2-(o-tolyl)-4,10-dihydropyrazolo[5,1-c][1,4]benzoxazepin-7-yl]acetamide,
N-[2-[2-(trifluoromethoxy)phenyl]-3-(trifluoromethyl)-4,10-dihydropyrazolo[5,1-c][1,4]benzoxazepin-7-yl]acetamide,
N-[2-[2-(trifluoromethoxy)phenyl]-3-(trifluoromethyl)-4,10-dihydropyrazolo[5,1-c][1,4]benzoxazepin-7-yl]cyclopropanecarboxamide,
N-[3-bromo-2-[2-(trifluoromethoxy)phenyl]-4,10-dihydropyrazolo[5,1-c][1,4]benzoxazepin-7-yl]propanamide,
N-[3-bromo-2-[2-(trifluoromethoxy)phenyl]-4,10-dihydropyrazolo[5,1-c][1,4]benzoxazepin-7-yl]-2-methyl-propanamide,
N-[3-bromo-2-[2-(trifluoromethoxy)phenyl]-4,10-dihydropyrazolo[5,1-c][1,4]benzoxazepin-7-yl]cyclopropanecarboxamide,
N-[3-methyl-2-(o-tolyl)-4,10-dihydropyrazolo[5,1-c][1,4]benzoxazepin-7-yl]cyclopropanecarboxam ide,
N-[3-bromo-2-(2-methoxyphenyl)-4,10-dihydropyrazolo[5,1-c][1,4]benzoxazepin-7-yl]-2-methyl-propanamide,
2-methyl-N-[3-methyl-2-(o-tolyl)-4,10-dihydropyrazolo[5,1-c][1,4]benzoxazepin-7-yl]propanamide,
N-[3-methyl-2-(o-tolyl)-4,10-dihydropyrazolo[5,1-c][1,4]benzoxazepin-7-yl]acetamide,
N-[3-bromo-2-(2-methoxyphenyl)-4,10-dihydropyrazolo[5,1-c][1,4]benzoxazepin-7-yl]cyclopropanecarboxamide,
N-[3-bromo-2-(o-tolyl)-4,10-dihydropyrazolo[5,1 -c][1,4]benzoxazepin-7-yl]-2-methyl-propanamide,
N-[3-fluoro-2-(o-tolyl)-4,10-dihydropyrazolo[5,1-c][1,4]benzoxazepin-7-yl]cyclopropanecarboxam ide,
N-[3-bromo-2-(o-tolyl)-4,10-dihydropyrazolo[5,1-c][1,4]benzoxazepin-7-yl]cyclopropanecarboxam ide,
N-[3-bromo-2-(o-tolyl)-4,10-dihydropyrazolo[5,1-c][1,4]benzoxazepin-7-yl]acetamide,
N-[3-bromo-2-(2-methoxyphenyl)-4,10-dihydropyrazolo[5,1-c][1,4]benzoxazepin-7-yl]acetamide,
N-[3-fluoro-2-(o-tolyl)-4,10-dihydropyrazolo[5,1-c][1,4]benzoxazepin-7-yl]acetamide,
2-methyl-N-[2-(o-tolyl)-4,10-dihydropyrazolo[5,1-c][1,4]benzoxazepin-7-yl]propanamide,
N-[2-(2-methoxyphenyl)-3-methyl-4,10-dihydropyrazolo[5,1-c][1,4]benzoxazepin-7-yl]acetamide,
N-[3,8-dichloro-2-(o-tolyl)-4,10-dihydropyrazolo[5,1 -c][1,4]benzoxazepin-7-yl]-2-methyl-propanamide,
N-(6-fluoro-2-(o-tolyl)-4H,10H-benzo[f]pyrazolo[5,1 -c][1,4]oxazepin-7-yl)propionamide
N-(2-(2-(trifluoromethyl)phenyl)-4H,10H-benzo[f]pyrazolo[5,1-c][1,4]oxazepin-7-yl)propionamide,
N-(2-(2-(trifluoromethyl)phenyl)-4H,10H-benzo[f]pyrazolo[5,1-c][1,4]oxazepin-7-yl)cyclopropanecarboxamide,
N-(2-(2,4-bis(trifluoromethyl)phenyl)-4H,10H-benzo[f]pyrazolo[5,1-c][1,4]oxazepin-7-yl)cyclopropanecarboxamide,
N-(2-(2-(trifluoromethyl)phenyl)-4H,10H-benzo[f]pyrazolo[5,1-c][1,4]oxazepin-7-yl)isobutyramide,
N-(2-(2,4-dimethylphenyl)-4H,10H-benzo[f]pyrazolo[5,1-c][1,4]oxazepin-7-yl)isobutyramide,
N-(2-(2-(trifluoromethyl)phenyl)-4H,10H-benzo[f]pyrazolo[5,1-c][1,4]oxazepin-7-yl)acetamide,
N-(2-(4-fluoro-2-methylphenyl)-4H,10H-benzo[f]pyrazolo[5,1-c][1,4]oxazepin-7-yl)propionamide,
N-(2-(5-fluoro-2-methylphenyl)-4H,10H-benzo[f]pyrazolo[5,1-c][1,4]oxazepin-7-yl)cyclopropanecarboxamide,
N-(2-(2-(trifluoromethoxy)phenyl)-4H,10H-benzo[f]pyrazolo[5,1-c][1,4]oxazepin-7-yl)propionamide,
N-(2-(2-(trifluoromethoxy)phenyl)-4H,10H-benzo[f]pyrazolo[5,1-c][1,4]oxazepin-7-yl)cyclopropanecarboxamide,
N-(2-(2-(trifluoromethoxy)phenyl)-4H,10H-benzo[f]pyrazolo[5,1-c][1,4]oxazepin-7-yl)isobutyramide,
N-(2-(2-(trifluoromethoxy)phenyl)-4H,10H-benzo[f]pyrazolo[5,1-c][1,4]oxazepin-7-yl)acetamide
N-(2-(o-tolyl)-4H,10H-benzo[f]pyrazolo[5,1-c][1,4]oxazepin-7-yl)cyclopropanecarboxamide.

7. A compound according to any of the claims 1 - 6 for use as a pharmaceutically active agent in medicine.

8. The compound according to any of the claims 1 - 6 for use in the treatment and/or prophylaxis of proliferative diseases, tumours, cancers, metastases, immunological disorders, autoimmune diseases, infectious diseases, inflammatory disorders and circulatory diseases.

9. The compound for use according to claim 8, wherein the proliferative disease, tumour, cancer or metastasis is selected from the group consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, estrogen dependent and independent breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's lymphomas), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarian carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma, tongue cancer, invasive ductal carcinoma, invasive lobular carcinoma, ductal carcinoma in situ, lobular carcinoma in situ, small-cell lung carcinoma, non-small-cell lung carcinoma, bronchial adenoma, pleuropulmonary blastoma, mesothelioma, brain stem glioma, hypophtalmic glioma, cerebellar astrocytoma, cerebral astrocytoma, neuroectodermal tumours, pineal tumors, sarcoma of the uterus, salivary gland cancers, anal gland adenocarcinomas, mast cell tumors, pelvis tumours, ureter tumours, hereditary papillary renal cancers, sporadic papillary renal cancers, intraocular melanoma, hepatocellular carcinoma (liver cell carcinomas with or without fibrolamellar variant), cholangiocarcinoma (intrahepatic bile duct carcinoma), mixed hepatocellular cholangiocarcinoma, squamous cell carcinoma, malignant melanoma, Merkel cell skin cancer, non-melanoma skin cancer, hypopharyngeal cancer, nasopharyngeal cancer, oropharyngeal cancer, oral cavity cancer, squamous cell cancer, oral melanoma, AIDS-related lymphoma, cutaneous T-cell lymphoma, lymphoma of the central nervous system, malignant fibrous histiocytoma, lymphosarcoma, rhabdomyosarcoma, malignant histiocytosis, fibrosarcoma, hemangiosarcoma, hemangiopericytoma, leiomyosarcoma., canine mammary carcinoma, and feline mammary carcinoma.

10. The compound for use according to claim 8, wherein the immunological disorder and/or autoimmune disease is selected from the group consisting of: asthma, diabetes, rheumatic diseases, AIDS, rejection of transplanted organs and tissues, rhinitis, chronic obstructive pulmonary diseases, osteoporosis, ulcerative colitis, sinusitis, lupus erythematosus, recurrent infections, atopic dermatitis / eczema and occupational allergies, food allergies, drug allergies, severe anaphylactic reactions, anaphylaxis, manifestations of allergic diseases, primary immunodeficiencies, antibody deficiency states, cell mediated immunodeficiencies, severe combined immunodeficiency, DiGeorge syndrome, Hyper-IgE syndrome, Wiskott-Aldrich syndrome, ataxia-telangiectasia, immune mediated cancers, white cell defects, autoimmune diseases, systemic lupus erythematosus, rheumatoid arthritis (RA), multiple sclerosis (MS), immune-mediated or Type 1 Diabetes Mellitus, immune mediated glomerulonephritis, scleroderma, pernicious anemia, alopecia, pemphigus, pemphigus vulgaris, myasthenia gravis, inflammatory bowel diseases, Crohn's disease, psoriasis, autoimmune thyroid diseases, Hashimoto's disease, dermatomyositis, goodpastture syndrome, myasthenia gravis pseudoparalytica, ophtalmia sympatica, phakogene uveitis, chronical agressive hepatitis, primary billiary cirrhosis, autoimunehemolytic anemy, and Werlof disease.

11. The compound for use according to claim 8, wherein the inflammatory disorder is selected from the group consisting of: abscessation, acanthameba, acanthamebiasis, acne vulgaris, actinomycosis, acute inflammatory dermatoses, acute laryngeal infections of adults, acute multifocal placoid pigmentary epitheliopathy, acute (thermal) injury, acute retinal necrosis, acute suppurative otitis media, algal disorders, allergic contact dermatitis, amyloidosis angioedema, ankylosing spondylitis, aspergillosis, atopic dermatitis, Aujeszky's disease, autoantibodies in vasculitis, babesiosis, bacterial disorders, bacterial laryngitis, bacterial meningitis, Behcet's disease, birdshot choroidopathy, blastomycosis, borna disease, brucellosis, bullous myringitis, bursitis, candidiasis, canine distemper encephalomyelitis, canine distemper encephalomyelitis in immature animals, canine ehrlichiosis, canine herpes virus encephalomyelitis, cholesteatoma, chronic (granulomatous) diseases, chronic inflammatory dermatoses, chronic relapsing encephalomyelitis, chronic suppurative otitis media, cicatricial pemphigoid, coccidiomycosis, coccidioidomycosis, common upper respiratory infection, contact ulcer and granuloma, Crohn's disease, cryptococcosis, cysticercosis, dermatomyositis, diphtheria, discoid lupus erythematosus, drug-induced vasculitis, drug or hypersensitivity reaction, encephalitozoonosis, eosinophilic meningoencephalitis, erythemal multiforme (EM minor), feline leukemia virus, feline immunodeficiency virus, feline infectious peritonitis, feline polioencephalomyelitis, feline spongiform encephalopathy, fibromyositis, Fuch's heterochromic cyclitis, gastroesophageal (laryngopharyngeal) reflux disease, giant cell arteritis, glanders, glaucomatocyclitic crisis, gonorrhea granular myringitis, granulomatous meningoencephalomyelitis, herpes simplex, histoplasmosis, idiopathic diseases, idiopathic inflammatory disorders, immune and idiopathic disorders, infections of the immunocompromised host, infectious canine hepatitis, inhalation laryngitis, interstitial nephritis, irritant contact dermatitis, juvenile rheumatoid arthritis, Kawasaki's disease, La Crosse virus encephalitis, laryngeal abscess, laryngotracheitis (croup), leishmaniasis, lens-induced uveitis, leprosy, leptospirosis, leukemia, lichen planus, lupus, lyme disease, lymphoma, meningitis, meningoencephalitis in greyhounds, miscellaneous meningitis / meningoencephalitis, microscopic polyangiitis, multifocal choroiditis, multifocal distemper encephalomyelitis in mature animals, multiple sclerosis, muscle tension dysphonias, mycotic (fungal) diseases, mycotic diseases of the CNS, necrotizing encephalitis, neosporosis, old dog encephalitis, onchocerciasis, parasitic encephalomyelitis, parasitic infections, pars planitis, parvovirus encephalitis, pediatric laryngitis, pollution and inhalant allergy, polymyositis, post-vaccinal canine distemper encephalitis, post-vaccinal rabies, prion protein induced diseases, protothecosis, protozoal encephalitis-encephalomyelitis, psoriasis, psoriatic arthritis, pug dog encephalitis, pyogranulomatous meningoencephalomyelitis, rabies, radiation injury, radiation laryngitis, radionecrosis, relapsing polychondritis, Reiters's syndrome, retinitis pigmentosa, retinoblastoma, rheumatoid arthritis, rickettsial disorders, rocky mountain spotted fever, salmon poisoning, sarcocystosis, sarcoidosis, schistosomiasis, scleroderma, scleroma, serpiginous choroiditis, shaker dog disease, Sjogren's syndrome, spasmodic croup, spirochetal (syphilis) diseases, spongiotic dermatitis, sporotrichosis, steroid responsive meningitis-arteritis, Stevens-Johnson syndrome (SJS, EM major), supraglottitis (epiglottitis), sympathetic ophthalmia, syngamus laryngeus, syphilis, systemic lupus erythematosus, systemic vasculitis in sarcoidosis, Takayasu's arteritis, tendinitis (tendonitis), thromboangiitis obliterans (Buerger's Disease), tick-borne encephalitis in dogs, toxic epidermal necrolysis (TEN), toxocariasis, toxoplasmosis, trauma, traumatic laryngitis, trichinosis, trypanosomiasis, tuberculosis, tularemia, ulcerative colitis, urticaria (hives), vasculitis, vasculitis and malignancy, vasculitis and rheumatoid arthritis, vasculitis in systemic lupus erythematosus, vasculitis in the idiopathic inflammatory myopathies, vasculitis of the central nervous system, vasculitis secondary to bacterial, fungal, and parasitic infection, viral disorders, viral laryngitis, vitiligo, vocal abuse, vocal-cord hemorrhage, Vogt Koyanagi Harada syndrome, Wegener's granulomatosis, Whipple's disease, pancreatitis, severe COVID-19, and Long Covid.

12. The compound for use according to claim 8, wherein the circulatory disease is selected from the group consisting of: high blood pressure, atherosclerosis, coronary artery disease, heart attack, heart failure, stroke, abdominal aortic aneurism, peripheral artery disease, myocardial infarction, mitral prolapse, stenosis, regurgitation, angina pectoris, arrhythmia, dysrhythmia and ischemia.

13. The compound for use according to claim 8, wherein the infectious disease is selected from the group consisting of: bacterial, parasitic, fungal or viral infectious diseases

14. A pharmaceutical composition comprising at least one compound according to any of the claims 1 - 6 as an active agent, together with at least one pharmaceutically acceptable carrier, excipient and/or diluent.

15. The pharmaceutical composition according to claim 14 for use in the treatment and/or prophylaxis of proliferative diseases, tumours, cancers, metastases, immunological disorders, autoimmune diseases, infectious diseases and inflammatory disorders.
